(19) Europäisches Patentamt
European Patent Office
Office européen des brevets

(11) Veröffentlichungsnummer: **0 503 471 A1**

(12) # EUROPÄISCHE PATENTANMELDUNG

(21) Anmeldenummer: **92103739.6**

(22) Anmeldetag: **05.03.92**

(51) Int. Cl.5: **C07D 495/14**, C07D 495/22, A61K 31/55, //(C07D495/14, 333:00,249:00,243:00), (C07D495/14,333:00,243:00, 235:00),(C07D495/22,333:00, 249:00,243:00,221:00)

(30) Priorität: **08.03.91 DE 4107521**

(43) Veröffentlichungstag der Anmeldung: **16.09.92 Patentblatt 92/38**

(84) Benannte Vertragsstaaten: **AT BE CH DE DK ES FR GB GR IT LI LU NL PT SE**

(71) Anmelder: **BOEHRINGER INGELHEIM KG**

**W-6507 Ingelheim am Rhein(DE)**
(84) **BE CH DE DK ES FR GR IT LI LU NL PT SE AT**

(71) Anmelder: **BOEHRINGER INGELHEIM INTERNATIONAL G.M.B.H.**

**W-6507 Ingelheim am Rhein(DE)**
(84) **GB**

(72) Erfinder: **Weber, Karl-Heinz, Dr.**
**Kaiser-Karl-Strasse 11**
**W-6535 Gau-Algesheim(DE)**
Erfinder: **Stransky, Werner, Dr.**
**Im Hippel 24**
**W-6535 Gau-Algesheim(DE)**
Erfinder: **Küfner-Mühl, Ulrike, Dr.**
**Schlossbergstrasse 8**
**W-6507 Ingelheim 4(DE)**
Erfinder: **Heuer, Hubert, Dr.**
**Am Sportfeld 74**
**W-6501 Schwabenheim(DE)**
Erfinder: **Birke, Franz, Dr.**
**Albrecht-Dürer-Strasse 23**
**W-6507 Ingelheim am Rhein(DE)**

(54) **Neue acylaminosubstituierte Hetrazepine.**

(57) Die Erfindung betrifft neue acylaminosubstituierte Hetrazepine, Verfahren zu ihrer Herstellung und ihre Verwendung als Arzneimittel.

EP 0 503 471 A1

Die Erfindung betrifft neue acylaminosubstituierte Hetrazepine, Verfahren zu ihrer Herstellung und ihre Verwendung als Arzneimittel.

Aus verschiedenen europäischen Patentanmeldungen sind Hetrazepine mit einer PAF-antagonistischen Wirkung bekannt, so z.B. aus den europäischen Patentanmeldungen 254 245, 328 924 und 407 955. Überraschenderweise wurde gefunden, daß Hetrazepine, die eine Acylaminogruppe als Strukturelement aufweisen, verbesserte pharmakologische Eigenschaften aufweisen.

Die neuen Hetrazepine entsprechen der allgemeinen Formel I,

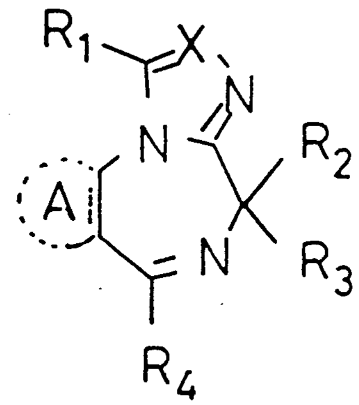

worin

- $R_1$ Wasserstoff, Halogen, eine verzweigte oder unverzweigte Alkylgruppe mit 1 bis 4 Kohlenstoffatom(en), bevorzugt Methyl, die gegebenenfalls durch Hydroxy oder Halogen substituiert sein kann, eine Cyclopropylgruppe, eine Cyclobutylgruppe;
- $R_2$ Wasserstoff, Methyl, Trifluormethyl oder Hydroxymethyl;
- $R_3$ Wasserstoff, Methyl, Trifluormethyl oder Hydroxymethyl;
- $R_4$ Phenyl, wobei der Phenylring ein- oder mehrfach, bevorzugt in 2-Stellung, durch Methyl, bevorzugt Halogen, besonders bevorzugt Chlor oder Brom, Nitro, Alkoxy, bevorzugt Methoxy und/oder Trifluormethyl substituiert sein kann, Pyridyl oder Thienyl, das gegebenenfalls durch $C_1$-$C_4$-Alkyl oder Halogen substituiert sein kann;
- X Stickstoff oder C-H;
- A ein Rest der Formel

a)

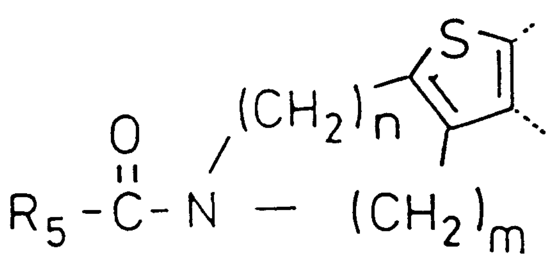

mit
n = 1, 2 oder 3
m = 1, 2 oder 3
und m + n = 2, 3 oder 4;

b)

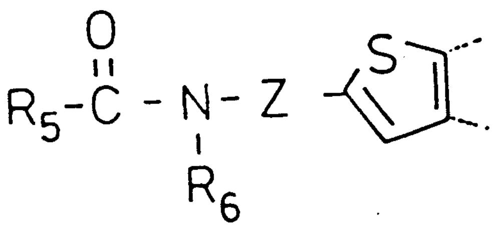

oder

c)

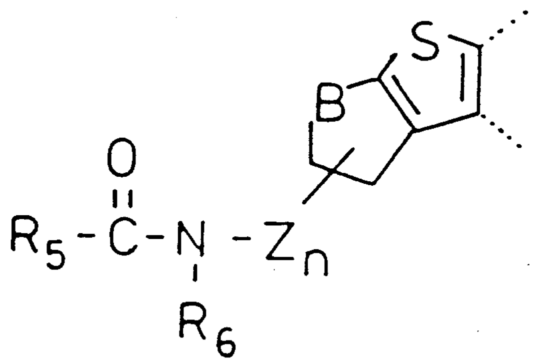

mit B = $CH_2$ oder $-CH_2-CH_2-$

worin

$R_5$ eine gegebenenfalls substituierte Alkylgruppe mit 1 bis 8, bevorzugt 1 - 4 C-Atomen, eine gegebenenfalls substituierte Arylgruppe, $CH_2$-Aryl, $CH_2$-$CH_2$-Aryl;

$R_6$ Wasserstoff oder eine gegebenenfalls substituierte Alkylgruppe mit 1 bis 8, bevorzugt 1 - 4 C-Atomen; eine gegebenenfalls substituierte Benzylgruppe und

Z eine Alkylgruppe mit 1 bis 8 C-Atomen;

$Z_n$ eine Alkylgruppe mit 1 bis 18 C-Atomen oder eine Einfachbindung,

bedeuten können,

gegebenenfalls in Form ihrer Racemate, ihrer Enantiomeren, ihrer Diastereomeren und ihrer Gemische.

Bevorzugt sind Hetrazepine der allgemeinen Formel I

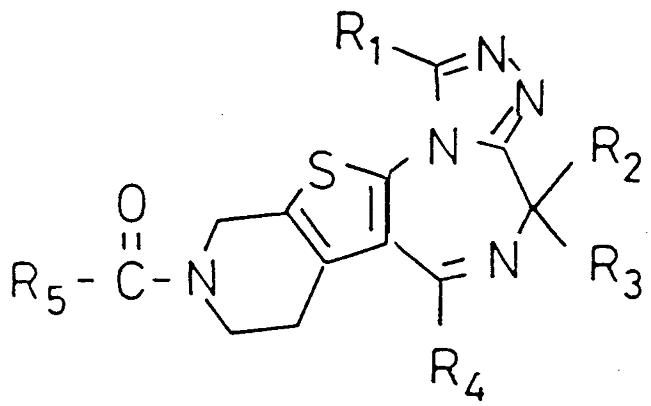

Ia

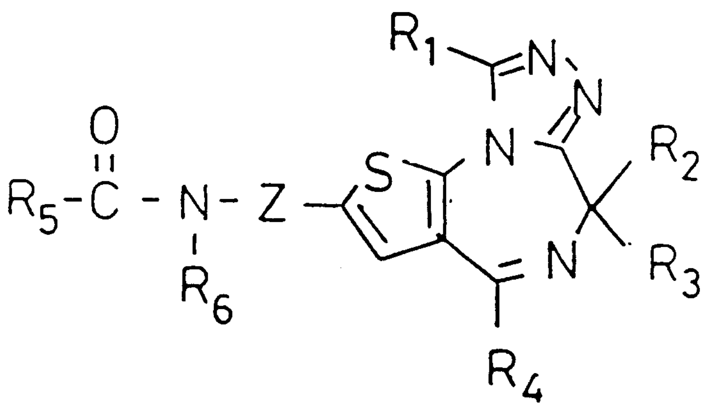

Ib

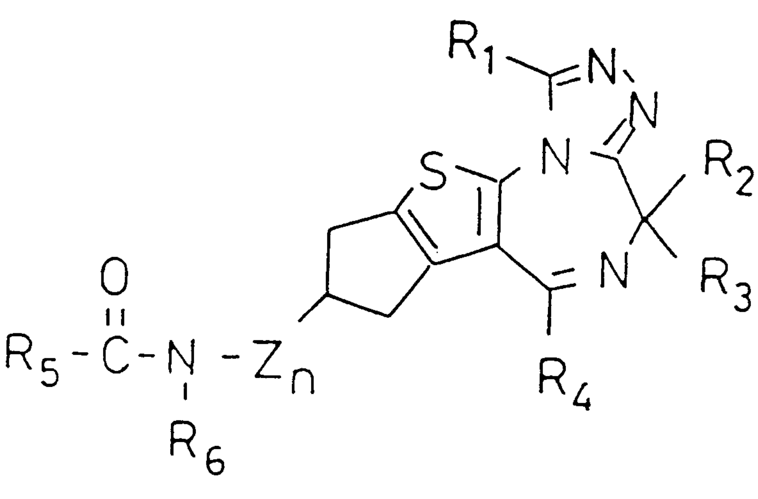

Ic

oder

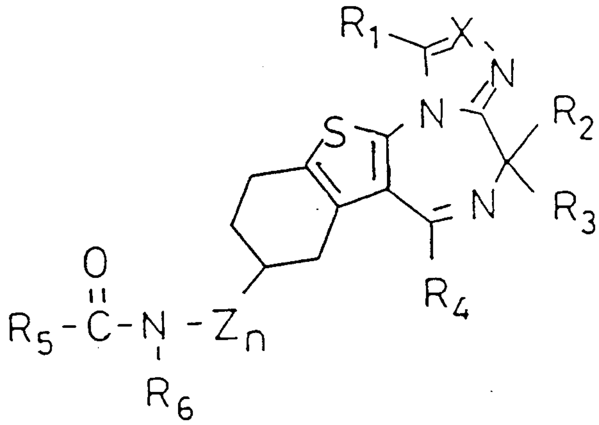

Id

worin

- $R_1$ $CH_3$;
- $R_2$ Wasserstoff oder Methyl;
- $R_3$ Wasserstoff oder Methyl;
- $R_4$ ortho-Chlorphenyl;
- $R_5$ $C_1$-$C_4$-Alkyl, gegebenenfalls substituiertes Phenyl, gegebenenfalls substituiertes -$CH_2$-Phenyl, gegebenenfalls substituiertes Thiophen, gegebenenfalls substituiertes Furan, gegebenenfalls substituiertes Pyridin;
- $R_6$ Wasserstoff oder $C_1$-$C_4$-Alkyl;
- Z eine lineare Alkylgruppe mit 1-4 C-Atomen, bevorzugt $CH_2$;
- $Z_n$ eine lineare Alkylgruppe mit 1-4 C-Atomen oder eine Einfachbindung;

bedeuten können, - gegebenenfalls in Form ihrer Racemate, ihrer Enantiomeren, ihrer Diastereomeren und ihrer Gemische.

Ganz besonders bevorzugt sind Verbindungen der allgemeinen Formel Ia

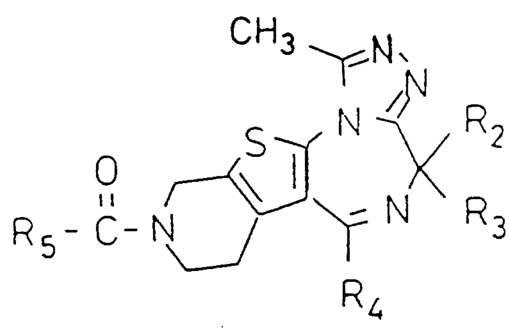

Ia

worin

- $R_2$ Wasserstoff oder Methyl;
- $R_3$ Wasserstoff;
- $R_4$ ortho-Chlorphenyl;
- $R_5$ einen Rest

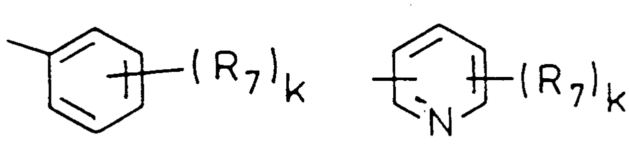

worin

- $R_7$ Wasserstoff, Halogen, Hydroxy, Nitro, Cyano, Trifluormethyl, gegebenenfalls durch Halogen oder Hydroxy substituiertes, verzweigtes oder unverzweigtes Alkyl mit 1 bis 8 - bevorzugt 1 bis 4 -

Kohlenstoffatom(en), gegebenenfalls durch Halogen oder Hydroxy substituiert, gegebenenfalls substituiertes $C_3$-$C_6$-Cycloalkyl,
gegebenenfalls substituiertes, unverzweigtes oder verzweigtes Alkoxy mit 1 bis 8 bevorzugt 1 bis 4 Kohlenstoffatom(en), $C_1$-$C_4$-Alkyl-S-, HS-,
Amino, $C_1$-$C_6$-Alkylamino,
$C_1$-$C_6$-Dialkylamino,
k = 1, 2 oder 3, wobei bei k > 1 $R_4$ gleich oder verschieden sein kann;

$R_5$ einen Rest

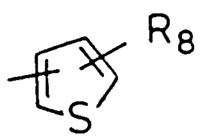

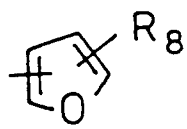

worin

$R_8$ Wasserstoff, Halogen oder $C_1$-$C_4$-Alkyl bedeuten können,

gegebenenfalls in Form ihrer Racemate, ihrer Entantiomeren, ihrer Diastereomeren und ihrer Gemische.

Bevorzugte Reste $R_5$ in der allgemeinen Formel Ia sind Reste der allgemeinen Formel

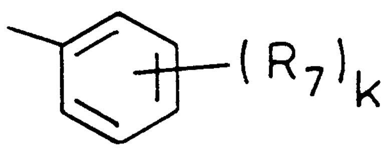

worin $R_7$ Wasserstoff, Halogen, Methyl, Ethyl, iso-Butyl, tert.-Butyl, Methoxy, bedeutet, besonders bevorzugt ist der Rest $R_7$ in der 4-Position des Phenylrings.

Die erfindungsgemäßen Verbindungen können im Diazepinring ein Asymmetriezentrum aufweisen, für den Fall, daß $R_2$ und $R_3$ verschieden substituiert sind.

Die bei der Synthese gegebenenfalls anfallenden Gemische optisch isomerer Verbindungen können durch Bildung von Diasteremoren sowie auch die nachfolgenden an sich bekannten Verfahren, wie z.B. durch Kristallisation oder durch chromatographische oder enzymatische Trennverfahren in die einzelnen optischen Isomeren getrennt werden. So zum Beispiel durch eine flüssigchromatographische Trennung, bei denen Cellulosetriacetat als stationäre Phase verwendet wird. Soweit nicht im einzelnen abweichende Angaben gemacht werden, werden die allgemeinen Definitionen im folgenden Sinn gebraucht:

Alkyl steht im allgemeinen für einen unverzweigten oder verzweigten Kohlenwasserstoffrest mit 1 bis 8 Kohlenstoffatom(en), der gegebenenfalls mit einem Halogenatom oder mehreren Halogenatomen - vorzugsweise Fluor - substituiert sein kann, die untereinander gleich oder verschieden sein können, wobei Niederalkylreste für einen verzweigten oder unverzweigten Kohlenwasserstoffrest mit 1 bis etwa 4 Kohlenstoffatom(en) bevorzugt sind. Unter Halogen versteht man die Atome Fluor, Chlor, Brom und Jod.

Als Alkylgruppen (auch soweit sie Bestandteile anderer Reste sind) werden, soweit nichts anderes angegeben ist, Methyl, Ethyl, Propyl, iso-Propyl, n-Propyl, n-Butyl, iso-Butyl, sek-Butyl und tert.-Butyl bevorzugt.

Erfindungsgemäß werden auch die den Acylaminorest ($R_5CONR_6$-) verbindenden Alkylengruppen Z und $Z_n$ als Alkylreste bezeichnet, um mißverständliche Interprepatation des Begriffs "Alkylen" zu vermeiden.

Cycloalkyl steht im allgemeinen für einen gesättigten oder ungesättigten cyclischen Kohlenwasserstoffrest mit 3 bis 6 Kohlenstoffatomen, der gegebenenfalls mit einem Halogenatom oder mehreren Halogenatomen einer Hydroxygruppe, einer Alkylgruppe, bevorzugt Methyl substituiert sein kann, die untereinander gleich oder verschieden sein können. Als Beispiele seien Cyclopropyl, Cyclobutyl, Cyclopentyl, Cyclopentenyl, Cyclohexyl, Cyclohexenyl genannt.

Phenylreste können beispielsweise mit einer oder mehreren Niederalkylgruppe(n), Alkoxygruppe(n), Nitrogruppe(n), Aminogruppe(n) und/oder einem oder mehreren Halogenatom(en) - untereinander gleich oder verschieden - substituiert sein.

Soweit nichts anderes angegeben, werden unter Arylgruppen gegebenenfalls ein- oder mehrfach substituierte aromatische Reste mit bis zu 10 Kohlenstoffatomen im Ringsystem verstanden, wie z.B. Phenyl, Pyridyl, Thienyl, Furyl oder Naphthyl, wobei der Phenylring bevorzugt ist. Als Substituenten

können, soweit im einzelnen nichts anderes angegeben ist, ein oder mehrere Atome aus der Gruppe Halogen, ein oder mehrere Reste aus der Gruppe Alkyl, Alkoxy, Amino, Alkyl- und Dialkylamino, Hydroxy vorliegen, wobei Methyl, iso-Butyl, Hydroxy oder Trifluormethyl bevorzugt sind.

Eine substituierte Phenylgruppe kann beispielsweise auch einen oder mehrere der nachfolgend genannten Substituenten tragen: $C_1$-$C_6$-Alkyl, $C_1$-$C_6$-Alkoxy, Halogen, Amino, Alkylamino, Dialkylamino, $CF_3$, $C_3$-$C_6$-Cycloalkyl, Cyano, $NO_2$, COH, COOH, $COOC_1$-$C_4$-Alkyl, Cyclopropyl, Hydroxy, SH, S-$C_1$-$C_4$-Alkyl, Hydroxymethyl.

Beispielhaft für substituiertes Phenyl werden genannt:

3-Chlorphenyl, 4-Chlorphenyl, 3-Bromphenyl, 4-Bromphenyl, 4-Fluormethyl, 2-Chlorphenyl, 2-Bromphenyl, 3-Fluorphenyl, 2,3-Dichlorphenyl, 2-Methylphenyl, 4-Methylphenyl, 3-Ethylphenyl, 4-Propylphenyl, 4-Isopropylphenyl, 4-Butylphenyl, 4-tert-Butylphenyl, 4-Iso-butylphenyl, 4-Pentylphenyl, 2,4-Dimethylphenyl, 2-Trifluormethylphenyl, 3-Trifluormethylphenyl, 4-Trifluormethylphenyl, 2-Methoxyphenyl, 4-Methoxyphenyl, 3-Ethoxyphenyl, 2-Propoxyphenyl, 4-Butoxyphenyl, 2,4-Dimethoxyphenyl, 3,4,5-Trimethoxyphenyl, 2,3,4,5,6-Pentafluorphenyl

Alkoxy steht im allgemeinen für einen über ein Sauerstoffatom gebundenen geradkettigen oder verzweigten Kohlenwasserstoffrest mit 1 bis 8 Kohlenstoffatomen. Besonders bevorzugt ist ein Alkoxyrest mit 1 bis 4 Kohlenstoffatomen. Als Beispiele seien Methoxy, Ethoxy, Propoxy, Isopropoxy, Butoxy, Isobutoxy, tert.-Butoxy, Pentoxy, Isopentoxy, Hexoxy, Isohexoxy, Heptoxy, Isoheptoxy, Octoxy oder Isooctoxy genannt.

PAF zeigt im pharmakologischen Experiment Bronchokonstriktion, Blutdrucksenkung, Auslösung einer Thrombozytenaggregation sowie eine proinflammatorische Wirkung.

Diese experimentell nachweisbaren Wirkungen des PAF weisen direkt oder indirekt auf mögliche Funktionen dieses Mediators in der Anaphylaxie, in der Pathophysiologie des Asthma bronchiale und allgemein in der Entzündung hin.

PAF-Antagonisten werden benötigt, um einerseits weitere pathophysiologische Funktionen dieses Mediators an Tier und Mensch aufzuklären und andererseits pathologische Zustände und Krankheiten, an denen PAF beteiligt ist, zu behandeln. Beispiele für die Indikationen eines PAF-Antagonisten sind Entzündungsprozesse des Tracheobronchialbaumes (z. B. akute und chronische Bronchitis, Asthma bronchiale) oder der Niere (z. B. Glomerulonephritis) oder der Gelenke (z. B. rheumatische Erkrankungen): anaphylaktische Zustände, Allergien und Entzündungen im Bereich der Schleimhäute (z.B. allergische Rhinitis) und der Haut (z.B. Psoriasis) sowie durch Sepsis, Endotoxine oder Verbrennungen bedingte Schockzustände. Weitere wichtige Indikationen für einen PAF-Antagonisten sind Läsionen und Entzündungen im Bereich der Magen- und Darmschleimhaut, wie z.B. Gastritis, im allgemeinen Ulcus pepticum, jedoch insbesondere Ulcus ventriculi und Ulcus duodeni; zur Behandlung von Thrombosen. Weiterhin erscheinen die erfindungsgemäßen Verbindungen zur Behandlung bei den folgenden Indikationsstellungen geeignet:

Obstruktive Lungenerkrankungen, bronchiale Hyperreaktivität; entzündliche Lungenwegserkrankungen, wie z.B. chronische Bronchitis; fibrotische Lungenwegserkrankungen, wie z.B. Fibrosing alveolitis und interstital pneumonitis syndrome; gynokologische Erkankungen z.B. Dysmennorhea, Schwangerschaftseklampsie, Schwangerschaftsbluthochdruck; Hemmung der Geburtswehen; Herz- Kreislauferkrankungen, wie z.B. Polytrauma, Anaphylaxie, Arteriosklerose; Krankheiten der fehlerhaften Blutgefäßbildung, z.B. fehlerhafte Blutgefäßbildung in den Augen; EPH-Gestose (edema-proteinuria Hypertension); entzündliche und immunologische Erkrankungen; Immunmodulation bei Transplantationen von Fremdgeweben, Immunmodulation bei Leukämie, Metastasenausbreitung z.B. bei bronchialer Neoplasie; weiterhin erweisen sich die erfindungsgemäßen Verbindungen als Cyto- und Organoprotektiv, z.B. zur Neuroprotektion, z.B. bei Leberzirrhose, z.B. Schutz bei Strahlenbehandlung, z.B. Anwendung während oder nach chirurgischen Eingriffen und in extra-Korporal Kreisläufen; DIC (disseminierte intravasale Gerinnung); zum Einsatz bei Organtransplantationen, zur Verminderung der Nebenwirkungen einer Arzneimitteltherapie, z.B. anaphylaktoide Kreislaufreaktionen, Kontrastmittelzwischenfälle, Nebenwirkungen bei der Tumortherapie; haemolytik uremic syndrome; heptarenal syndrome, Unverträglichkeiten bei Bluttransfusionen; Leberversagen (z.B. Hepatitis, primär biliar zirrhosis, primär sclerosende cholangitis), Vergiftungen, z.B. Amanitaphalloides-Intoxikation [Knollenblätterpilzvergiftung];

Symptome von parasitären Erkrankungen (z.B. Wurmerkrankungen); Autoimmunerkrankungen. Weiterhin sind folgende Indikationen von Interesse: Immunfunktion bei Aids, Diabetes, juvenile Diabetes, diabetische Retinopathie, polytraumatischer Schock, hämorrhagischer Schock, Erkrankungen des ZNS, z.B. Migräne, Agoraphobie (panic disorder): Ischämie, Multiple Sklerose; entzündliche Darmerkrankungen; Colitis Ulcerosa, Morbus Crohn: pulmonaler Hochdruck sowie chronische ischämische Herzinsuffizienz, PAF Antagonisten der allgemeinen Formel I eignen sich zur Behandlung von pathologischen Blutgasveränderungen, wie beispielsweise respiratorische Acidose, metabolische Alkalose. Allein oder in Kombination mit Anticholinergica können PAF-Antagonisten zur Verbesserung der Blutgaswerte bei Phosphorsäureestervergiftungen

eingesetzt werden. Es ist bekannt, daß PAF-Antagonisten allein - oder in Kombination mit immunsuppressiv wirkenden Verbindungen (z.B. Cyclosporine oder FK-506) zur Behandlung von Asthma, Autoimmunerkrankungen und bei Transplantationen eingesetzt werden können, wobei nicht nur eine Verbesserung der Wirksamkeit aber auch eine Verminderung der Toxizität von Cyclosporinen oder FK-506 zu erwarten sind. Weiterhin wird vorgeschlagen, PAF-Antagonisten in Kombination mit Antihistaminica zu verwenden. Bezüglich der Definition der Antihistaminica wird inhaltlich auf die Europäische Patentanmeldung 345 731 Bezug genommen. Ferner ist bekannt, daß PAF-Antagonisten in Kombination mit $\beta_2$-Mimetica zur Behandlung des Asthma bronchiale eingesetzt werden können.

PAF assoziierte Interaktion mit Gewebshormon (autocoid hormones), Lymphokine und anderen Mediatoren sind bekannt. PAF-Antagonisten eignen sich auch zur Behandlung des durch verminderte $\beta$-Rezeptorstimulation am Herzen bzw. durch Down Regulation der $\beta$-Rezeptoren am Herzen verursachten Krankheiten, z.B. zur Behandlung der zu geringen Pumpleistung des Herzens bei akutem Herversagen, beispielsweise nach Myocardinfarkt und cardiogenem Schock, oder bei chronischen cardivasculären Erkrankungen wie kongestive Herzinsuffizienz, Herzinsuffizienz nach Myocardinfarkt oder bei ischämischen Cardiomyopathien.

Sie können auch in Kombination eingesetzt werden, vor allem bei solchen Indikationen, für die PAF-Antagonisten geeignet sind. Dementsprechend können die PAF-Antagonisten z.B. mit $\beta$-Adrenergika, Parasympatholytika, Corticosteroiden, Antiallergika, Sekretolytika, Antibiotika kombiniert werden. Bei der Kombination mit TNF (Tumor-Nekrose-Faktor) wird eine bessere Verträglichkeit (Ausschaltung störender Nebenwirkungen) des TNF erreicht; TNF läßt sich daher gewünschtenfalls auch in höheren Dosen einsetzen als bei seiner alleinigen Anwendung.

(Unter "Kombination" ist hier auch die Anwendung der beiden Wirkstoffe in getrennten Zubereitungen und in einem gewissen zeitlichen Abstand zu verstehen). Bei der gemeinsamen Anwendung der erfindungsgemäßen Verbindungen mit $\beta$-Adrenergika kann ein synergistischer Effekt erzielt werden, z.B. in der Broncholyse. Sehr vorteilhaft ist auch die Kombination der PAF-Antagonisten mit Immunsuppressiva, z.B. den verschiedenen Cyclosporinen.

Wässerige Lösungen, die einen der erfindungsgemäßen Verbindungen enthalten, eignen sich zur Aufbewahrung von Organen, die zur Transplantation bestimmt sind. PAF-Antagonisten eignen sich als Zusatz zu Blut- und Plasma-Konserven.

Die neuen Verbindungen können topisch, oral, parenteral oder durch Inhalation verbreicht werden. Die Verbindungen liegen hierbei als aktive Bestandteile in üblichen Darreichungsformen vor, z.B. in Zusammensetzungen, die im wesentlichen aus einem inerten pharmazeutischen Träger und einer effektiven Dosis des Wirkstoffes bestehen, wie z.B. Tabletten, Dragées, Kapseln, Oblaten, Pulver, Lösungen, Suspensionen, Inhalationsaerosole, Salben, Emulsionen, Siruppe, Suppositorien.

Die therapeutischen und prophylaktische Dosis ist abhängig von der Beschaffenheit und Ernsthaftigkeit des Krankheitszustandes. Eine wirksame Dosis der erfindungsgemäßen Verbindungen liegt bei oraler Anwendung zwischen 1 und 200, vorzugsweise zwischen 10 und 80 mg/Dosis, bei intravenöser oder intramuskulärer Anwendung zwischen 0,001 und 50, vorzugsweise zwischen 0,1 und 30 mg/Dosis. Für die Inhalation sollen Lösungen, die 0,01 bis 1,0, vorzugsweise 0,1 bis 0,5 % Wirkstoff enthalen, eingesetzt werden, ferner Pulver und Suspensionen in verflüssigten Treibgasen.

Die neuen Hetrazepine sind sehr starke PAF-Antagonisten und anderen bekannten diazepinoiden PAF-Antagonisten in folgenden Kriterien überlegen:

- es besteht eine totale Dissoziation zwischen dem PAF-Antagonismus und den Benzodiazpin-Receptor vermittelten Effekten;
- überlegende Bindungsaffinität zum PAF-Rezeptor auf gewaschenen Humanplättchen und zeigen eine stärkere Hemmung der PAF-induzierten Plättchenaggregation;
- Darüber hinaus hemmen sie in einer überlegenen Weise die PAF-(30 ng/kg x min) induzierte Bronchokonstriktion nach oraler und parenteraler Applikation am Meerschweinchen in Kombination mit einer sehr langen Wirkdauer (größer 15 Std. nach oraler Applikation am Meerschweinchen).

Die Hemmung der PAF-induzierten Plättchenaggregatien kann nach folgender Methodik bestimmt werden.

Gesunden männlichen und weiblichen Spendern im Alter von 18 bis 35 Jahren, die mehrere Tage vor Blutentnahme keine Medikamente (Aspirin oder andere nicht steroidale Entzündungshemmer) eingenommen hatten, wurden jeweils 200 ml Blut aus einer nicht gestauten Vene mit Hilfe einer Plastikspritze, in der sich 3.8 %-ige Natriumcitratlösung befand, entnommen. Das Verhältnis Natriumcitratlösung: Blut betrug 1:9. Das Citratblut wurde in Plastikröhrchen bei 150 x g ( = 1200 U/min) und Raumtemperatur 20 min lang zentrifugiert (Heraeus Christ Tischzentrifuge 124).

Die Messung der Thrombozytenaggregation in vitro erfolgte nach der Methode von Born und Cross (1963), wobei dem TRP unter ständigem Rühren ein Aggregationsauslöser (PAF) zugesetzt wurde. Zur

Messung wurde in 1 ml Plastikküvetten, die jeweils einen kleinen Metallstift (Rührer, 1000 U/min) enthielten, 0.8 ml TRP und 0.2 ml modifizierte Tyrode-Lösung (s. unten) gegeben. Die Prüfsubstanz wurde 2 bis 3 min vor Auslösung der Aggregation in einem Volumen von 10 µl zugesetzt. Als Lösungsmittel dienten entweder DMSO und Wasser oder eine verdünnte HCl-Lösung. Die Kontrollansätze enthielten das entsprechende Volumen dieser Lösungsmittel. Nach Registrierung der Ausgangsabsorption (2-3 min) wurde die Aggregation induziert. In einem Volumen von 10 µl erfolgte die Gabe von PAF ($5 \times 10^{-8}$ M; Bachem Feinchemikalien) in die Küvette.

Die modifizierte Tyrode-Lösung hatte folgende Zusammensetzung: 136.9 mM NaCl; 2.68 mM KCl; 0.5 mM $MgCl_2$; 1.8 mM $CaCl_2$; 0.42 mM $NaH_2PO_4$; 5.55 mM Glucose und 11.9 mM $NaHCO_3$.

Zur Beurteilung von Substanzeffekten wurde das Maximum der ersten Aggregationswelle verwendet. Die durch den Aggregationsauslöser induzierte maximale Absorption (= maximale Aggregation = 100 %) wurde gleichzeitig in einem Parallelansatz (im 2. Kanal des Aggregometers) zu jedem Testansatz mitgeführt und als 100 % Wert verwendet. Der unter der Wirkung der Testsubstanz erreichte Aggregationswert wurde als % des Kontrollwertes (-ansatz) angegeben. Mit Hilfe dieses Verfahrens wurden Konzentrationswirkungskurven mit einem Stichprobenumfang von jeweils n = 4 erstellt und $IK_{50}$-Werte (Konzentration bei einer 50 %igen Aggregationshemmung) berechnet.

[$^3$H]PAF-Rezeptor-Bindung an vitalen humanen Blutplättchen

Bestimmt wird die konkurrierende Wechselwirkung von Prüfsubstanzen (hier PAF-Antagonisten) mit der bekannten Wechselwirkung des Radioliganden [$^3$H]PAF zum gleichen Rezeptor.

Die Bindungsstudien wurden an vitalem menschlichen Thrombozyten vorgenommen. Blutproben von gesunden Spendern wurden mit ACD-Puffer verdünnt und zentrifugiert (15 Min., 160 x g). Das blättchenreiche Plasma wird durch Chromatographie an Sepharose CL-2B [Zum Eluieren: HEPES-Puffer, pH 7,4 20°C] gereinigt.

Definierte Mengen [z.B. 800 µl] der Blättchen- suspension wurden 90 Min. bei Raumtemperatur inkubiert, d.h. vermischt mit:

a) einer 30 pikomolarem [$^3$H]PAF-Lösung, verdünnt mit Puffer.

b) mit einer 30 pikomolaren [$^3$H] PAF-Lösung, die gleichzeitig eine µ-molare (nicht markierte) PAF-Lösung enthält,

c) mit der 30 pikomolaren [$^3$H]PAF-Lösung und den Lösungen der Prüfsubstanzen (unterschiedliche Konzentrationen)

a) Dient zur Ermittlung der totalen Bindung,

b) Dient zur Ermittlung der unspezifischen Bindung.

Die Reaktion wurde durch Vakuumfiltration abgestoppt. Die Filter mit den Blutplättchen werden mit Scintilationsflüssigkeit versetzt und die verbliebene Radioaktivität in einem Counter gemessen.

Die spezifische Bindung ergibt sich aus totaler Bindung minus unspezifischer Bindung. Man bestimmt entweder die $IC_{50}$-Werte (d.h. jene Konzentration der Prüfsubstanz, die 50 % des Radioliganden - hier [$^3$H] PAF - vom Rezeptor verdrängt), und gibt diese an.

Oder errechnet hieraus die $K_i$-Werte. Dies kann computerunterstützt durch ein Iterationsverfahren der Bindungskurven geschehen. $IC_{50}$- bzw. Ki-Werte sind ein Maß für die Rezeptoraffinität der Testsubstanz. Kleinere Werte zeigen die höhere Affinität an.

| Beispiel | [$^3$H]PAF-Bindung Ki[nM] |
|---|---|
| 1 | 2.9 |
| 1a ((-) Isomer) | 1.9 |
| 1b ((+) Isomer) | 71.0 |
| 2 | 1.8 |
| 20 | 2.2 |
| 25 | 2.4 |
| 82 | 2.5 |
| 67 | 2.8 |
| 29 | 7.8 |
| 73 | 10.0 |

Im Vergleich zu dem aus dem Stand der Technik (EP 367 110) bekannten 3-Cyclopropylcarbonyl-6-(2-chlorphenyl)-11-methyl-2,3,4,5-tetrahydro-4H-pyrido[4,3 : 4',5']thieno[3,2-f][1,2,4]triazolo[4,3]-a]-1,4-diazepin,

das einen Bindungswert von Ki = 13 [nMol] aufweist, zeigen die erfindungsgemäß beanspruchten Verbindungen eine deutliche Überlegenheit auf.

Die Herstellung der erfindungsgemäßen Verbindungen kann in Analogieverfahren gemäß dem Stand der Technik erfolgen.

Die erfindungsgemäßen Hetrazepine der allgemeinen Formel I können nach an sich bekannten Methoden hergestellt werden.

Syntheseverfahren A:

Verbindungen der allgemeinen Formel Ia, die einen ankondensierten stickstoffhaltigen Heterocyclus aufweisen, sind beispielsweise durch Umsetzung der entsprechend substituierten Säurehalogenide - insbesondere Säurechloride - der allgemeinen Formel II

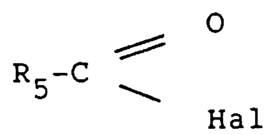

mit Verbindungen der allgemeinen Formel III

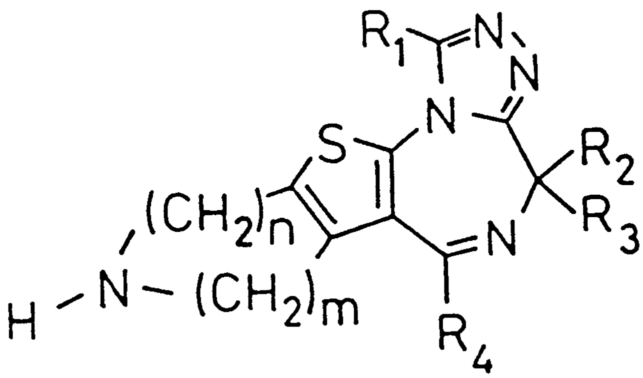

zugänglich. Analoge Reaktionen sind beispielsweise in der europäischen Patentanmeldung 367 110 beschrieben, auf die hiermit inhaltlich Bezug genommen wird.

Die Umsetzung der Reaktanden kann beispielsweise in inerten organischen Lösungsmitteln - wie z.B. Dichlormethan, Chloroform, in Dialkylethern, z.B. Diethylether, tert.-Butyl-methylether, Tetrahydrofuran, Aceton, Methylethylketon, Benzol, Toluol oder Dimethylformamid - durchgeführt werden, vorzugweise in Gegenwart einer organischen oder anorganischen Base, wie z.B. Triethylamin, Pyridine, Natriumhydrogencarbonat, Kaliumcarbonat, Natriumcarbonat. Wird anstelle des Säurechlorids der Formel II die freie Säure oder ein geeignetes Säureanhydrid eingesetzt, so erfolgt die Umsetzung vorzugsweise in Gegenwart 1,1-Carbonyldiimidazol, Dicyclohexylcarbodiimid oder eines anderen Kondensationsreagenzes.

Alternativ können die Verbindungen der allgemeinen Formel Ia nach folendem Synthesekonzept hergestellt werden.

($R_4$ hat bevorzugt die Bedeutung ortho-Chlorphenyl, $R_5$ hat bevorzugt die Bedeutung Methyl)

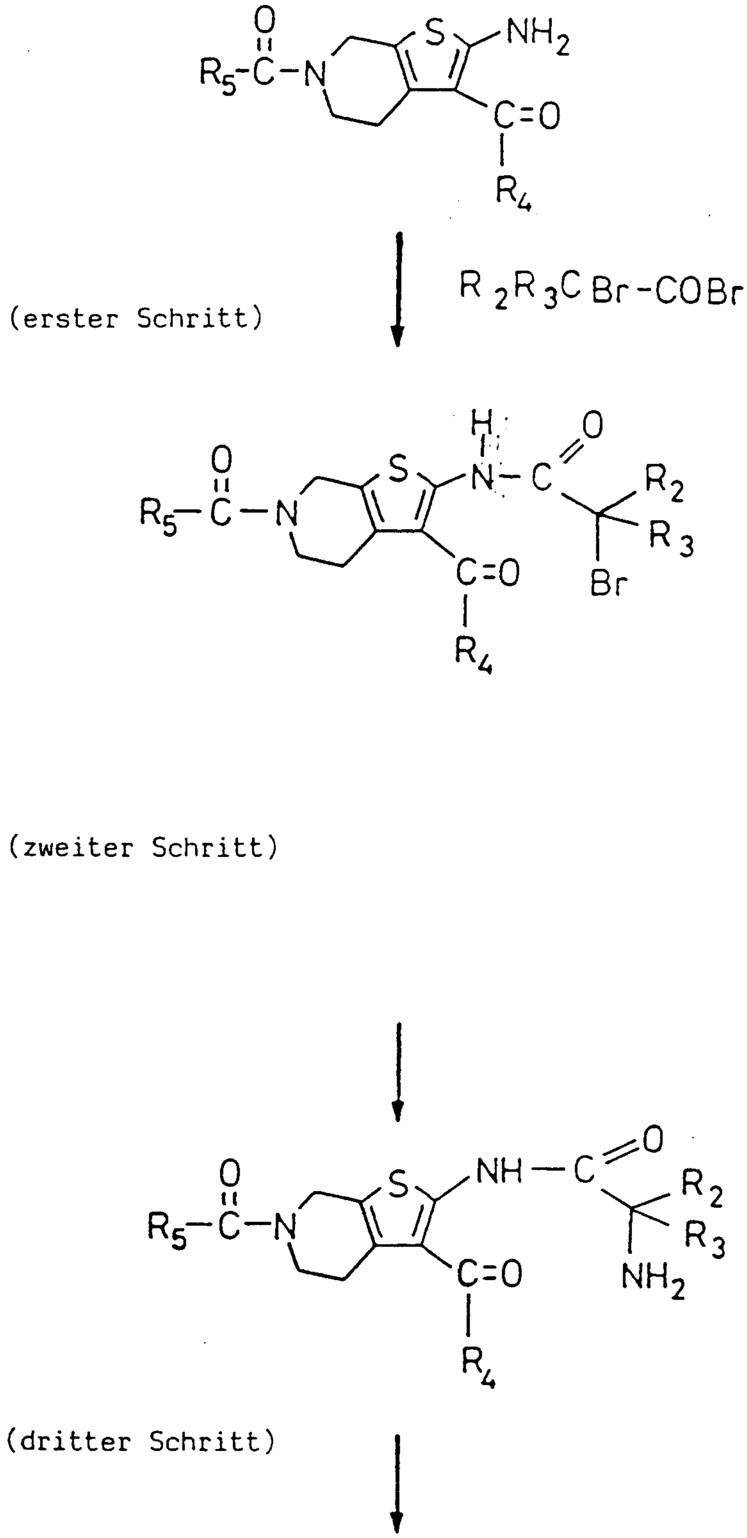
R 2R 3C Br-COBr
(erster Schritt)
(zweiter Schritt)
(dritter Schritt)

IV

V

VI

VII

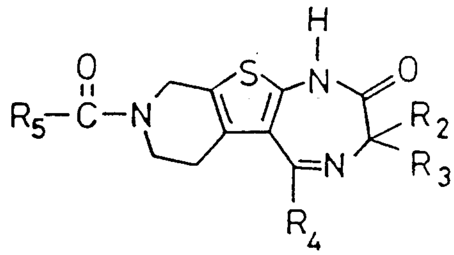

VIII

(vierter Schritt)

$P_2S_5$

IX

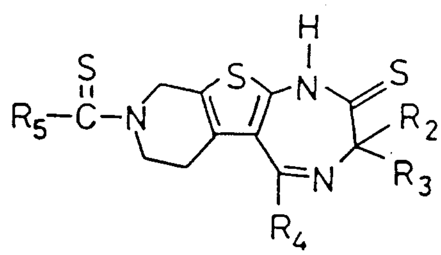

(fünfter Schritt)

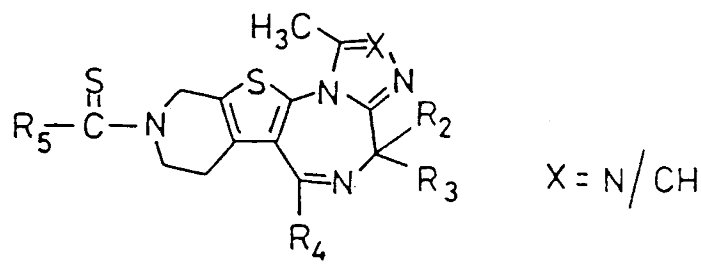

X

(sechster Schritt)

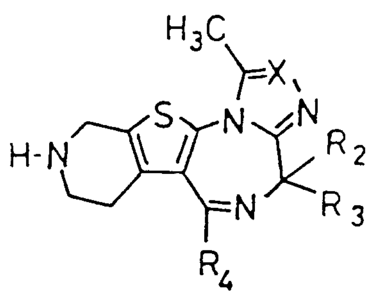

XI

1. Stufe:

Aminothiophene der allgemeinen Formel IV werden mit Verbindungen der allgemeinen Formel V unter an sich bekannten Bedingungen einer Kondensationsreaktion unterworfen. woraus Verbindungen des Typs

VI resultieren.

Beispielsweise in einer Zweiphasen-Reaktion eines organischen Lösunsmittels, wie z.B. Toluol, Benzol, Xylol in Gegenwart einer Base, wie z.B. Alkalihydroxide, wie Natriumhydroxid, Kaliumhydroxid oder in Gegenwart von Natriumhydrogencarbonat oder Kaliumhydrogencarbonat. Die Umsetzung kann auch in Gegenwart eines inerten Lösungsmittels, wie z.B. Dichlormethan, Dichlorethan, Tetrahydrofuran, Toluol, Benzol, Xylol oder Dimethylformamid in Gegenwart einer Base ausgewählt aus der Gruppe - Natriumhydoxid, Kaliumhydroxid, eines Alkalihydrides, wie z.B. Natriumhydrid oder Kaliumhydrid - erfolgen.

Sind $R_2$ und $R_3$ = Wasserstoff, so erfolgt die Umsetzung auf dieser Stufe bevorzugt mit Chloressigsäurechlorid, ist $R_2$ = Methyl und $R_3$ Wasserstoff, ist das bevorzugte Reagenz V 2-Brompropionsäurebromid, im Fall von $R_2$ und $R_3$ = Methyl erfolgt die Umsetzung mit 2-Brom-2-methylpropionsäurebromid.

2. Stufe

Auf dieser Stufe erfolgt die Aminierung mittels gasförmigen Ammoniaks. Die Umsetzung erfolgt in einem Temperaturbereich zwischen 30° und 100°C, entweder durch direkte Umsetzung der Reaktanden oder in Gegenwart eines inerten organischen Lösungsmittels, wie z.B. Tetrahydrofuran, Dioxan, Ethylacetat, Chloroform, Dichlormethan, Methanol, Ethanol oder Pyridin.

3. Stufe

Die Cyclisierung zu dem Diazepinon der allgemeinen Formel VIII erfolgt nach an sich bekannten Verfahren, so z.B. in einem inerten Lösungsmittel in Gegenwart eines sauren Katalysators, wie z.B. Essigsäure oder Kieselgel unter azeotroper Entfernung des bei der Cyclisierung gebildeten Wassers.

4. Stufe

Die Überführung in das Thion IX erfolgt mit Phosphorpentasulfid unter herkömmlichen Methoden.

5. Stufe

Diese Reaktionsstufe beinhaltet den Aufbau des anellierten Triazolo- oder Imidazolorestes.

Hierzu kann eine Verbindung der allgemeinen Fomel IX mit einem Säurehalogenid, oder aber mit Hydrazin und anschließend mit einem Säurehalogenid (bevorzugt einem Säurechlorid), oder aber mit einem Orthoester ($R_1$-$C(OCH_3)_3$) umgesetzt werden.

Die Umsetzung des Thions mit einem Säurehalogenid erfolgt in einem inerten organischen Lösungsmittel, wie z.B. Dioxan, Dimethylformamid, Tetrahydrofuran oder einem geeigneten Kohlenwasserstoff, wie z.B. Benzol oder Toluol bei Temperaturen zwischen Raumtemperatur und dem Siedepunkt des Reaktionsgemisches. Die Isolierung der Endprodukte geschieht nach bekannten Methoden, wie z.B. durch Kristallisation.

Die Umsetzung des Thions IX mit Hydrazin erfolgt in inerten organischen Lösungsmitteln, wie beispielsweise Tetrahydrofuran, Dioxan, halogenierten Kohlenwasserstoffen, wie z.B. Methylenchlorid oder in geeigneten Kohlenwasserstoffen, bei Temperaturen zwischen Raumtemperatur und dem Siedepunkt des Reaktionsgemisches.

Die weitere Umsetzung mit einem Säurehalogenid IX oder einem Orthoester erfolgt in einem inerten organischen Lösungsmittel, wie z.B. halogenierten Kohlenwasserstoffen, cyclischen oder offenkettigen aliphatischen Ethern, kann aber auch direkt in Substanz erfolgen. Die Isolierung des Endprodukts IA erfolgt nach bekannten Methoden, beispielsweise durch Kristallisation.

Ein Verfahren zur Herstellung der Imidazo-anellierten Hetrazepine (X = CH) besteht in der Umsetzung des Thions IX mit einem α-Aminoaldehyd-alkylacetal oder mit einem α-Aminoketon-alkylketal der allgemeinen Formel

$$H_2NCH_2CR_1(OCH_3)_2$$

wobei $R_1$ Wasserstoff oder eine Alkylgruppe mit 1 bis 4 Kohlenstoffatomen oder eine Cyclopropylgruppe bedeutet.

Analogieverfahren zur Synthese eines Acetals oder Ketals sowie ein Analogieverfahren zum Ringschluß sind in der Schweizerischen Patentschrift Nr. 580 099 beschrieben.

6. Stufe

Die Abspaltung der Schutzgruppe erfolgt durch Hydrolyse nach an sich bekannten Methoden, z.B. durch Erhitzen in Gegenwart von Kaliumhydroxid, Natriumhydroxid, Natriumethanolat, Natriummethoxid, Kaliumethanolat, Natriummethanolat, Natriumethanolat oder Kaliummethanolat.

Syntheseverfahren B:

Verbindungen der allgemeinen Formel I, worin A einen der Reste

b) 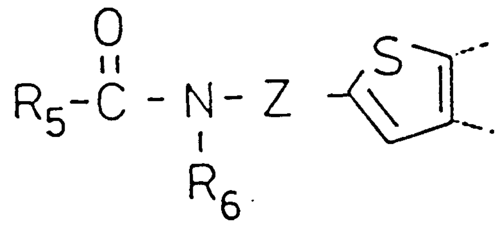

oder

c) 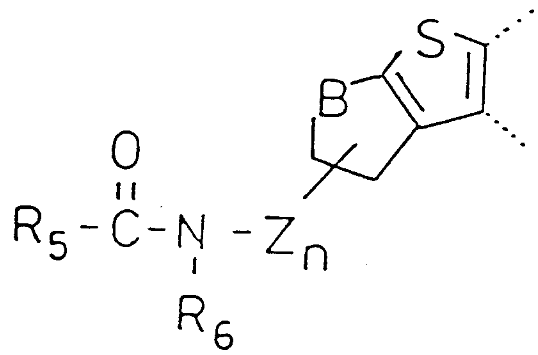

bedeuten können in Analogieverfahren durch Umsetzung der geeigneten Amine der Formel I mit A gleich

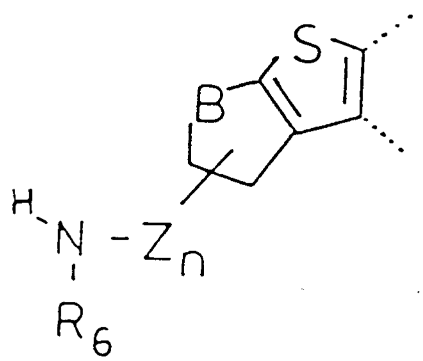

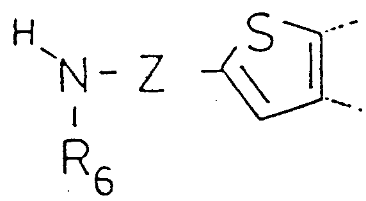

mit reaktiven Säurederivaten - insbesondere Säurehalogeniden der Formel $R_5$CO-Halogen - erhalten werden. Die Umsetzung kann in inerten organischen Lösungsmitteln bei erhöhter Temperatur durchgeführt werden.

Die Synthese oben genannter Amine erfolgt beispielsweise in Analogie zu den in den in den europäischen Patentanmeldungen 230 942 und 254 245 beschriebenen.

Beispiel 1

3-(4-Chlorbenzoyl)-6-(2-chlorphenyl)-8,11-dimethyl-2,3,4,5-tetrahydro-4H-pyrido[4,3 : 4',5']thieno[3,2-f]-[1,2,4]-triazolo[4,3-a]-1,4-diazepin

a) 4-Chlorbenzoylpiperidon

40,0 g (0.26 mol) 4-Piperidonhydrochlorid (x $H_2O$) und 38,6 g Kaliumcarbonat werden in 300 ml Tetrahydrofuran suspendiert. Unter Rühren fügt man 45,4 g 4-Chlorbenzoylchlorid hinzu und läßt 2 Stunden bei Raumtemperatur und 2 Stunden unter Rückfluß nachreagieren. Das Reaktionsgemisch wird im Vakuum eingedampft, der Rückstand mit 2N Salzsäure neutralisiert und mit Dichlormethan extrahiert. Aus der organischen Phase erhält man nach dem Entfernen des Extraktionsmittel 38 g eines zähen Öles.

$^1$H-NMR ($CDCl_3$): $\delta$ = 7.40 (4H, s, aryl-H); 3.84 (4H, m, N-$(CH_2)_2$; 2.50 (4H, m, $(CH_2)_2C=O$).

b) 2-Amino-3-(2-chlorbenzoyl)-6(4-chlorbenzoyl)-tetrahydropyrido[2,3-c]-thiophen

Eine Mischung von 16 g (70 mmol) Benzoylpiperidon, 12,7 g o-Chlorcyanoacetophenon und 2,3 g Schwefel in 50 ml Dimethylformamid werden mit 5 ml Triethylamin versetzt und 2 Stunden bei 60 - 70 ° C gerührt. Man dampft das Reaktionsgemisch im Vakuum ein und nimmt den Rückstand in 100 ml Essigester auf.

Anschließend wird mit Wasser gewaschen, die organische Phase abgetrennt, getrocknet und eingedampft. Der Rückstand kristallisiert aus einer Essigester/Ether-Mischung zur Kristallisation gebracht. Man erhält 17 - 18 g der Titelverbindung in Form gelber Kristalle vom Fp. 208-210 ° C.

$^1$H-NMR ($CDCl_3$): $\delta$ = 7.68 - 7.19 (10 H, m, aryl-H, $NH_2$); 4.45 (2H, m, N-$CH_2$); 3.51 (2H, m, N-$CH_2\underline{CH_2}$-); 1.81 (2H, m, N-$CH_2\underline{CH_2}$-).

c) 2-(2-Brompropionylamino)-3(2-chlorbenzoyl)-6-(4-chlorbenzoyl)-tetrahydropyrido[2,3-c]-thiophen

17,0 g (39 m mol) Aminoketon werden in 170 ml Dichlormethan gelöst, zunächst mit 4.1 ml Pyridin und anschließend tropfenweise unter Rühren mit 9.3 g 2-Brompropionsäurechlorid versetzt, wobei man die Temperatur bei 20-25 ° C hält. Man rührt 1 Stunde bei Raumtemperatur nach, dampft das Reaktionsemisch im Vakuum ein und chromatographiert den Rückstand an $SiO_2$ (Dichlormethan/Methanol 99 : 1). Aus dem Eluat erhält man nach dem Entfernen des Elutionsmittels 18 g der Titelverbindung in Form von Kristallen vom Fp. 180 - 182 ° C.

$^1$H-NMR ($CDCl_3$): $\delta$ = 12.80 (1H, s, NH-C=O); 7.57 - 7.19 (8H, m, aryl-H); 4.62 (2H, m, N-$CH_2$); 4.67 (1H, qu, J = 7Hz, CH-$CH_3$); 3.56 (2H, m, N-$\underline{CH_2}CH_2$), 1.99 (3H, d, J = 7Hz, CH-$\underline{CH_3}$); 1.94 (2H, m, N-$CH_2CH_2$-).

d) 2-(2-Aminopropionylamino)-3-(2-chlorbenzoyl)-6-(4-chlorbenzoyl)-tetrahydropyrido[2,3-c]thiophen

18,0 g Aminopropionylketon werden in einem Autoklaven mit 20 ml Essigester, 15 ml Dichlorethan und 2 g flüssigem Ammoniak 1 Stunde bei 12 - 13 bar auf 100 ° C erhitzt. Man dampft den Rückstand ein, nimmt in Dichlormethan auf, wäscht mit Wasser und trocknet die organische Phase nach der Phasentrennung. Nach dem Abdestilllieren des Lösungsmitels verbleiben 16 g der Titelverbindung in Form eines amorphen Rückstandes, der direkt weiter eingesetzt werden kann.

e) 3-(4-Chlorbenzoyl)-6-(2-chlorphenyl)-8-methyl-2,3,4,5-tetrahydro-4H-pyrido[4,3 : 4',5']thieno[3,2-f][1,4]-diazepin-9-on

16 g rohes Aminopropionylaminoketon werden mit 250 ml Toluol und 100 g Kieselgel versetzt und am Wasserabscheider 2 Stunden auf Rückflußtemperatur erhitzt. Nach dem Erkalten wird abgesaugt und der Rückstand dreimal mit jeweils 200 ml siedendem Methanol extrahiert. Die Methanolauszüge werden vereinigt, eingedampft und der Rückstand an $SiO_2$ chromatographiert (Dichlormethan). Nach dem Eindampfen erhält man durch dem Kristallisation aus einer Essigester/Ether-Mischung 6 g der Titelverbindung in Form von Kristallen vom Fp. 258 - 260 ° C.

f) 3-(4-Chlorbenzoyl)-6-(2-chlorphenyl)-8-methyl-2,3,4,5-tetrahydro-4H-pyrido[4,3 : 4',5']thieno[3,2-f][1,4]-diazepin-9-thion

(10 mmol) des nach dem oben beschriebenen Verfahren hergestelltn Diazepinons, 50 ml Diaethylenglykoldimethylether (Diglyme) und 1,7 g Natriumhydrogencarbonat werden unter Rühren mit 2,5 g Phosphorpentasulfid versetzt und 1,5 Stunden bei 65 - 70 ° C gerührt. Man gießt das Reaktionsgemisch auf Eiswasser, saugt ab und nimmt die Kristalle in einer Dichlormethan/Methanol-Mischung auf. Nach wiederholter Filtration wird die Mischung eingedampft, das verbliebene Wasser mit Toluol azeotrop abdestilliert und der Rückstand über $SiO_2$ (Methanol/Dichlormethan) chromatographiert. Aus dem Eluat erhält man 3.1 g der Titelverbindung in Form gelber Kristalle, die bei 238 ° C schmelzen.

3 g dieses Thions werden in 30 ml Tetrahydrofuran gelöst und mit 0.32 g Hydrazinhydrat versetzt. Man rührt 30 Minuten bei Raumtemperatur nach, dampft i. Vak. ein und versetzt den Rückstand mit Ether, wobei dessen Kristallisation eintritt. Man erhält 2,2 g Kristalle vom Fp. 170 - 175 ° C. Diese werden in 20 ml Alkohol und 1,2 ml Orthoessigsäureethylester 1 Stunde unter Rückfluß erhitzt. Man dampft das Reaktionsgemisch im Vakuum ein und chromatographiert den Rückstand. Man erhält 2,2 g der Titelverbindung als farblose Kristalle vom Fp. 269 - 271 ° C.

Die gleiche Verbindung wird erhalten, wenn man 4,5 g 3-(4-Chlorbenzoyl)-6-(2-chlorphenyl)-8-methyl-2,3,4,5-tetrahydro-4H-pyrido[4,3 : 4',5']thieno[3,2-f]-1,4-diazepin-9-on mit 80 ml absolutem Dioxan und

10,5 g Phosphorpentachlorid 1 Stunde bei Raumtemperatur rührt, das Reaktionsgemisch (Imidchlorid) mit 15 g Essigsäurehydrazid versetzt und 30 Minuten weiterrührt. Man dampft vorsichtig ein, versetzt den Rückstand mit Wasser, extrahiert das Produkt mit Dichlormethan und erhält aus den vereinigten Eluaten die offenkettige Verbindung in Form tiefroter Kristalle, die durch Erhitzen mit 10 g $SiO_2$ in 50 ml Toluol zur Tielverbindung cyclisiert werden.
$^1$H-NMR (DMSO-d6): δ = 7.65 - 7.19 (8H, m, aryl-H); 5.03, 4.67 (2H, m, N-$CH_2$); 4.34 (1H, qu, J = 7 Hz, CH-$CH_3$); 4.05, 3.36 (2H, m, N-$\underline{CH_2}CH_2$-); 2.59 (3H, s, triazol-$CH_3$); 2.19, 1.50 (2H, m, N-$CH_2\underline{CH_2}$); 1.88 (3H, d, J = 7Hz, -CH-$\underline{CH_3}$).

Enantiomerentrennung mit Hilfe einer chiralen Säule

2 g des Racemates werden in 25 ml einer Mischung aus Cyclohexan und Dioxan 1 : 1 gelöst (Ultraschallbad). Diese Lösung gibt man auf eine Chirasphor-Säule der Firma E. Merck, Darmstadt (Korngröße 5 µm) und eluiert im Recycling-Betrieb mit Cyclohexan/Dioxan. Nach vollständiger Enantiomerentrennung werden die Lösungen präparativ aufgearbeitet. Man erhält als erstes Eluat das Isomer (1a) mit $[\alpha]_D^{20}$ + 36,8 (Methanol) und aus dem zweiten Eluat das Isomer (1b) mit $[\alpha]_D^{20}$ -36,8° (Methanol).

Beispiel 2

3-(4-Isobutylbenzoyl)-6-(2-chlorphenyl)-8,11-dimethyl-2,3,4,5-tetrahydro-4H-pyrido[4,3 : 4',5']thieno-[3,2-f]-[1,2,4]triazolo[4,3-a]1,4diazepin

a) Ausgehend von N-Acetylpiridon wird analog Beispiel 1 zunächst das 3-Acetyl-6-(2-chlorphenyl)-8-methyl-2,3,4,5-tetrahydro-4H-pyrido[4,3 : 4,5][3,2-f]-1,4-diazepin-9-on aufgebaut. Hieraus erhält man das 3-Thioacetyl-6-(2-chlorphenyl)-8,11-dimethyl-2,3,4,5-tetrahydro-4H-pyrido[4,3 : 4',5']thieno-[3,2-f]-1,2,4-triazolo[4,3-a]- 1,4-diazepin in Form von Kristallen vom Fp. 290 - 291°C.
$^1$H-NMR ($CDCl_3$): δ = 7.53 - 7.10 (8H, m, aryl-H); 5.06, 4.67 (2H, m, N-$CH_2$-); 4.30 (1H, qu, J = 7Hz, CH-$CH_3$); 3.64, 3.39 (2H, m, N-$\underline{CH_2}CH_2$-); 2.68 (3H, s, $CH_3$-triazol); 2.50 (2H, d, J = 6Hz, $\underline{CH_2}$-CH); 2.21, 1.87 (2H, m, N-$CH_2\underline{CH_2}$-); 2.12 (3H, d, J = 7Hz, CH-$\underline{CH_3}$); 1.89 (1H, m, $\underline{CH}(CH_3)_2$); 0.90 (6H, d, J = 7Hz, $CH(\underline{CH_3})_2$).
Analog zu dem von uns in der europäischen Patentanmeldung 204 245 früher beschriebenen Verfahren erhält man hieraus durch Verseifung mit methanolischer Kalilauge die Nor-Verbindung 6-(2-Chlorphenyl)-8,11-dimethyl-2,3,4,5-tetrahydro-4H-pyrido[4,3:4',5']thieno[3,2-f]-1,2,4-triazolo[4,3-a]-1,4-diazepin als Kristalle vom Fp. 205 - 207°C.
15 g (39 mmol) dieser Norverbindung werden in 250 ml gelöst bzw. suspendiert und mit 5,9 Dichlormethan/Triethylamin versetzt. Unter Rühren und Eiskühlung tropft man bei 10 - 15°C 7,7 g 4-Isobutylbenzoylchlorid hinzu und läßt 30 Minuten nachrühren. Man wäscht mit Wasser, trennt die organische Phase ab, dampft teilweise ein und chromatographiert den Rückstand an $SiO_2$ - (Dichlormethan/Methanol 97 : 3). Nach dem Kristallisieren aus einer Dichlormethan/Ether-Mischung erhält man 12,5 g der Titelverbindung in Form von Kristallen vom Fp. 248 - 250°C. Das 4-Isobutylbenzoylchlorid kann wie auf Seite 34 dargestellt, synthetisiert werden.
$^1$H-NMR ($CDCl_3$): δ = 7.56 - 7.20 (4H, m, aryl-H); 5.07, 4.47 (2H, m, N-$CH_2$); 4.30 (1H, qu, J = 7Hz, CH-$CH_3$); 3.75, 3.40 (2H, m, N-$\underline{CH_2}CH_2$); 2.74 (1H, m, CH-C=O); 2.69 (3H, s, $CH_3$-triazol); 2.19, 1.74 (2H, m, N-$CH_2\underline{CH_2}$); 2.13 (3H, d, J = 7Hz, CH-$\underline{CH_3}$); 1.11 (6H, d, -$CH(CH_3)_2$).
b) 4-Isobutylbenzaldehyd
134 g Isobutylbenzol werden im Autoklaven mit 206 g Benzol und 145,5 g Aluminiumchlorid versetzt und mit trockenen HCl-Gas gesättigt (2-3 bar). Anschließend drückt man Kohlenmonoxid auf, bis ein Druck von 35-40 bar erreicht ist und rührt 4 Stunden bei 25°C nach. Das Reaktionsgemisch wird auf 1 kg Eis gegossen, die organische Phase abgetrennt und mit verdünnter Salzsäure, Wasser und Natriumhydrogencarbonat-Lösung gewaschen und anschließend eingedampft. Der Rückstand wird destilliert. Man erhält aus der Hauptfraktion ($Kp_{15}$: 122 - 125°C) 100 g der Titelverbindung entsprechend 61.6 % d.Th.
$^1$H-NMR ($CDCl_3$): δ = 9.99 (1H, s, CH=O); 7.77, 7.28 (4H, 2d, J = 9Hz, aryl-H); 2.55 (2H, d, J = 6Hz, $\underline{CH_2}$-CH); 1.90 (1H, m, $CH_2$-$\underline{CH}$-); 0.92 (6H, d, J = 7Hz, $CH(\underline{CH_3})_2$).
c) 4-Isobutylbenzoesäure
39 g des nach dem vorstehend beschriebenen Verfahren hergestellten Aldehyds werden mit 560 ml 2N Natronlauge versetzt. Unter kräftigem Rühren werden allmählich 24 g Kaliumpermanganat zugegeben. Man rührt ca. 12 Stunden bei Raumtemperatur nach, saugt über Kieselgur ab und säuert das Filtrat mit

konz. Salzsäure an. Die dabei ausfallenden Kristalle werden abgesaugt und getrocknet. Man erhält Ausbeute 16 - 17 g der Titelverbindung als Kristalle vom Fp. 138 - 140 ° C.

d) 4-Isobtuylbenzoylchlorid

16,2 g der nach dem zuvor beschriebenen Verfahren hergestellten Carbonsäure werden mit 50 ml Benzol und 7,5 ml reinem Thionylchlorid 4 Stunden unter Rückfluß gerührt. Nach dem Entfernen des Lösungsmittels wird der Rückstand im Vakuum ($Kp_{18}$: 139 - 141 ° C) destilliert. Man erhält die Titelverbindung in einer Ausbeute 12 bis 13 g.

Beispiel 3

6-(2-Chlorphenyl)-4H-1-methyl-8-[N-(4-chlorphenylcarbonyl)-N-methyl-3-aminopropyl]-thieno[3.2-f]-[1.2.4]-triazolo[4.3-a][1.4]diazepin

6-(2-Chlorphenyl)-4H-8-[2-(methoxycarbonyl)-ethyl]-1-methylthieno[3.2-f][1.2.4]triazolo[4.3-a][1.4]-diazepin wird in dem System $NaBH_4$/tert.-Butanol/Methanol insgesamt 4 - 6 Stunden zum Rückfluß erhitzt, die Vollständigkeit der Reduktion mittels Dünnschichtchromatographie (Kieselgel, Eluens Methylenchlorid/Methanol (9 : 1) kontrolliert, der entstandene Alkohol aus Aceton umkristallisiert, anschließend in Dichlormethan mit Methansulfonsäurechlorid/Triethylamin in das entsprechende Mesylat überführt. Dieses wird innerhalb einer Reaktionszeit von 3 Stunden bei 120 ° C und einem Druck von 11.4 bar mit Methylamin zur Reaktion gebracht und das so erhaltene N-Methyl-3-aminopropyl-Derivat in Pyridin/Dioxan mittels 4-Chlorbenzoylchlorid in 50 %-iger Ausbeute in das gewünschte Amid umgewandelt, das als hellgelbes amorphes Pulver anfällt.

$^1$H-NMR ($CDCl_3$);

$\delta$ = 7.52 - 7.19 (8H, m, Aryl-H); 6.43 (1H, s, Thiophen-H); 4.92 (2H, s, $CH_2$-7-Ring); 3.86 (2H, m, N-$CH_2$); 2.86 (3H, s, N-$CH_3$); 2.84 (2H, m, N-$CH_2$-$CH_2$-$CH_2$-); 2.69 (3H, s, $CH_3$-Triazol); 1.99 (2H, m, N-$CH_2$-$CH_2$-$CH_2$-).

Beispiel 4

6-(2-Chlorphenyl)-4H-1-methyl-8-[N-(4-chlorphenylcarbonyl)-N-ethyl-aminomethyl]-thieno[3.2-f][1.2.4]-triazolo[4.3-a][1.4]diazepin

Ausgehend von 6-(2-Chlorphenyl)-8-formyl-4H-1-methylthieno[3.2-f][1.2.4]triazolo[4.3-a][1.4]diazepin und Ethylamin erhält man durch reduktive Aminierung in Methanol als Solvens und Raney-Nickel als Katalysator unter quantitativer Wasserstoffaufnahme bei Raumtemperatur innehalb einer Reaktionszeit von 3 Stunden die Zwischenverbindung 6-(2-Chlorphenyl)-4H-1-methyl-8-(N-ethyl)- aminomethylthieno[3.2-f][1.2.4]triazolo-[4.3-a][1.4]diazepin in Form von Kristallen vom Fp. 153 - 155 ° (Diisopropylether) in 43 %-iger Ausbeute.

Die Zwischenverbindung wird anschließend in Pyridin/Dioxan-Mischung mit 4-Chlor-benzoylchlorid umgesetzt und das Reaktionsprodukt mittels Flash-Chromatographie an Kieselgel mit Dichlormethan/Methanol (9 : 1) als Eluens gereinigt. Man erhält die Titelverbindung als hellgelbes amorphes Pulver in 65 %-iger Ausbeute.

$^1$H-NMR ($CDCl_3$):

$\delta$ = 7.50 - 7.22 (8H, m, Aryl-H); 6.61 (1H, s, Thiophen-H); 4.95 (2H, s, $CH_2$-7-Ring); 4.68 (2H, s, N-$CH_2$); 3.30 (2H, m, N-$CH_2$-$CH_3$); 2.74 (3H, s, $CH_3$-Triazol); 1.15 (3H, t, J = 7Hz, N-$CH_2$-$CH_3$

In Analogie zu dem allgemein beschriebenen Syntheseverfahren sowie zu den Beispielen 1 und 2 können folgende Verbindungen der allgemeinen Formel Ia

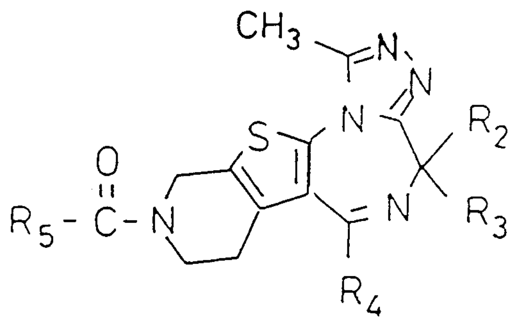

Ia

mit $R_4$ gleich ortho-Chlorphenyl und $R_2$, $R_3$ und $R_5$ wie in der Tabelle angegeben, synthetisiert werden.

| Nr. | $R_5$ | $R_2$ | $R_3$ | Fp°C |
|---|---|---|---|---|
| 1 | 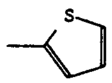 | H | H | |
| 2 | " | H | $CH_3$ | NMR 256-257 |
| 3 | 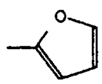 | H | $CH_3$ | NMR 253-255 |
| 4 | " | H | H | |
| 5 | 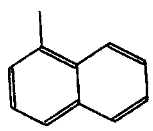 | H | $CH_3$ | |
| 6 | " | H | H | |
| 7 | 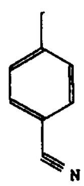 | H | $CH_3$ | 260-262 |
| 8 | " | H | H | |
| 9 | 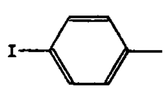 | H | $CH_3$ | |
| 10 | 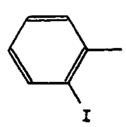 | H | H | |
| 11 | 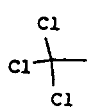 | H | $CH_3$ | |

| Nr. | $R_5$ | $R_2$ | $R_3$ | Fp°C |
|---|---|---|---|---|
| 12 | 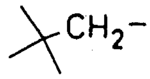 | H | $CH_3$ | |
| 13 | 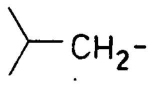 | H | H | |
| 14 | $Cl-CH_2-$ | H | $CH_3$ | |
| 15 | 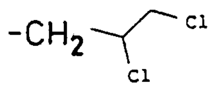 | H | H | |
| 16 | 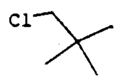 | H | $CH_3$ | |
| 17 | 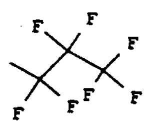 | H | H | |
| 18 | 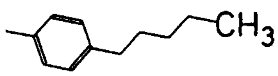 | H | $CH_3$ | |
| 19 | 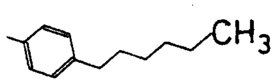 | H | H | |
| 20 | 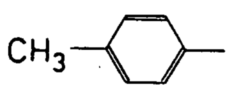 | H | $CH_3$ | 258-259°C |
| 21 | 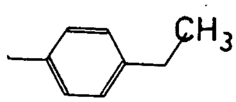 | H | H | |
| 22 | 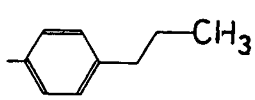 | H | $CH_3$ | |

| Nr. | $R_5$ | $R_2$ | $R_3$ | Fp°C |
|---|---|---|---|---|
| 23 | 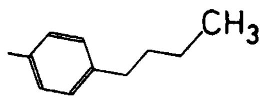 | H | $CH_3$ | 245-247 |
| 24 | 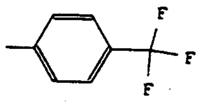 | H | H | |
| 25 | 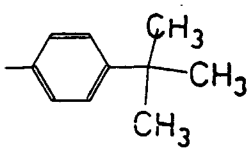 | H | $CH_3$ | 188-190°C |
| 26 | '' | H | H | |
| 27 | 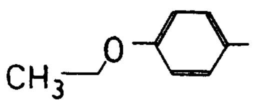 | H | $CH_3$ | |
| 28 | 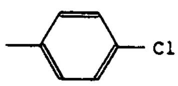 | H | H | |
| 29 | 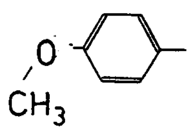 | H | $CH_3$ | 210-212°C |
| 30 | 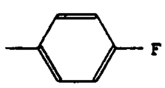 | H | H | |
| 31 | 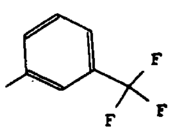 | H | $CH_3$ | |
| 32 | 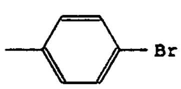 | H | H | |
| 33 | 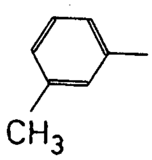 | H | $CH_3$ | |

| Nr. | $R_5$ | $R_2$ | $R_3$ | Fp°C |
|---|---|---|---|---|
| 34 | 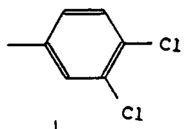 | H | $CH_3$ | |
| 35 | 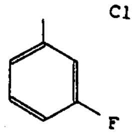 | H | H | |
| 36 | 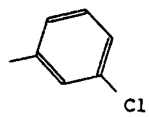 | H | $CH_3$ | |
| 37 | 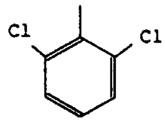 | H | H | |
| 38 | 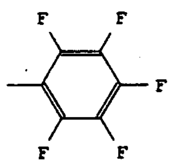 | H | $CH_3$ | 225-227 |
| 39 | " | H | H | |
| 40 | 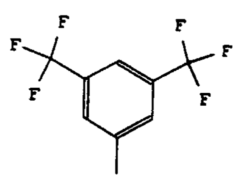 | H | $CH_3$ | |
| 41 | " | H | H | |
| 42 | 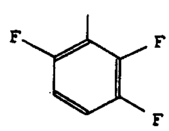 | H | $CH_3$ | |
| 43 | 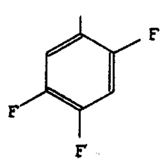 | H | H | |
| 44 | 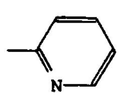 | H | $CH_3$ | |

| Nr. | $R_5$ | $R_2$ | $R_3$ | Fp°C |
|---|---|---|---|---|
| 45 | 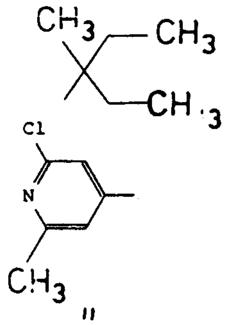 | H | $CH_3$ | |
| 46 | | H | H | |
| 47 | ″ | H | $CH_3$ | |
| 48 | 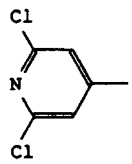 | H | H | |
| 49 | 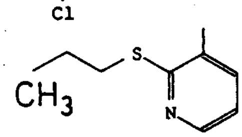 | H | $CH_3$ | |
| 50 | 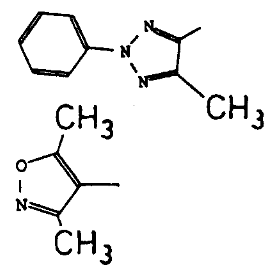 | H | H | |
| 51 | | H | $CH_3$ | |
| 52 | 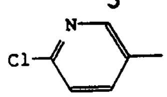 | H | H | |
| 53 | 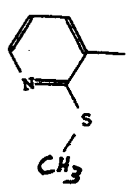 | H | $CH_3$ | |
| 54 | 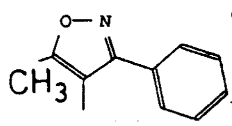 | H | H | |
| 55 | 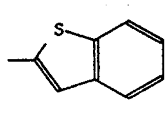 | H | $CH_3$ | |

| Nr. | $R_5$ | $R_2$ | $R_3$ | Fp°C |
|---|---|---|---|---|
| 56 | 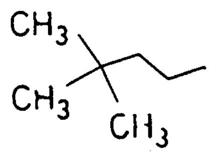 | H | $CH_3$ | |
| 57 | " | H | H | |
| 58 | 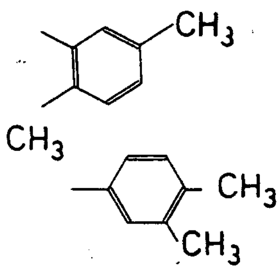 | H | $CH_3$ | |
| 59 | | H | H | |
| 60 | 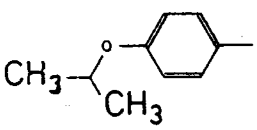 | H | $CH_3$ | |
| 61 | 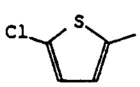 | H | H | |
| 62 | 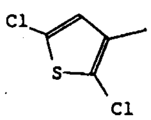 | H | $CH_3$ | |
| 63 | " | H | H | |
| 64 | 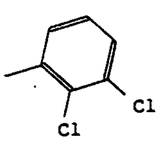 | H | $CH_3$ | |
| 65 | 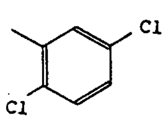 | H | H | |
| 66 | 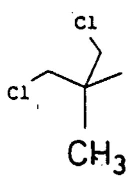 | H | $CH_3$ | |

| Nr. | $R_5$ | $R_2$ | $R_3$ | Fp°C |
|---|---|---|---|---|
| 67 | 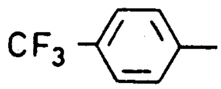 | H | $CH_3$ | 305-307°C |
| 68 | " | H | H | |
| 69 | " | $CH_3$ | $CH_3$ | |
| 70 | 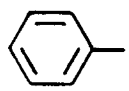 | H | H | |
| 71 | " | H | $CH_3$ | 248-250°C |
| 72 | " | $CH_3$ | $CH_3$ | |
| 73 | $(CH_3)_3C$- | H | $CH_3$ | 255-257°C |
| 74 | " | H | H | |
| 75 | " | $CH_3$ | $CH_3$ | |
| 76 | $(CH_3)_2CH$- | $CH_3$ | $CH_3$ | |
| 77 | " | H | $CH_3$ | 225-226°C |

| Nr. | $R_5$ | $R_2$ | $R_3$ | Fp°C |
|---|---|---|---|---|
| 78 | 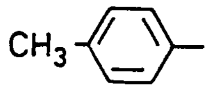 | $CH_3$ | $CH_3$ | |
| 79 | " | H | H | |
| 80 | 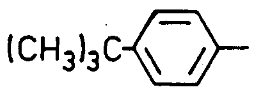 | $CH_3$ | $CH_3$ | |
| 81 | 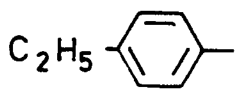 | H | H | |
| 82 | " | H | $CH_3$ | 255-256°C |
| 83 | " | $CH_3$ | $CH_3$ | |
| 84 | 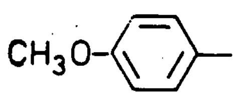 | $CH_3$ | $CH_3$ | |
| 85 | " | H | H | |
| 86 | 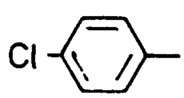 | H | $CH_3$ | NMR |
| 87 | " | H | H | 178 - 180 |
| 88 | " | $CH_3$ | $CH_3$ | 242 - 247 |

| Nr. | $R_5$ | $R_2$ | $R_3$ | Fp°C |
|---|---|---|---|---|
| 89 | N≡C- 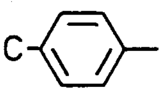 | H | $CH_3$ | NMR 260-262 |
| 90 | " | H | H | |
| 91 | " | $CH_3$ | $CH_3$ | |
| 92 | $(CH_3)_2$ CH-$CH_2$- 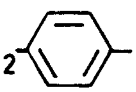 | H | H | 242-243 |
| 93 | " | H | $CH_3$ | NMR 245-247 |
| 94 | " | $CH_3$ | $CH_3$ | |
| 95 | 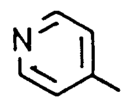 | $CH_3$ | $CH_3$ | |
| 96 | 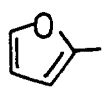 | $CH_3$ | $CH_3$ | |
| 97 | $F_3C$- 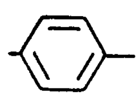 | H | $CH_3$ | NMR 305-307 |
| 98 | " | $CH_3$ | $CH_3$ | |
| 99 | 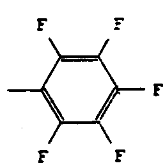 | $CH_3$ | $CH_3$ | |

| Nr. | $R_5$ | $R_2$ | $R_3$ | Fp°C |
|---|---|---|---|---|
| 100 | 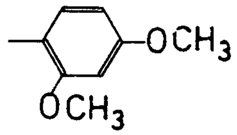 | H | $CH_3$ | |
| 101 | 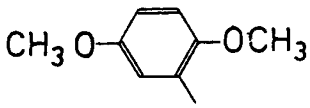 | H | H | |
| 102 | 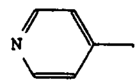 | H | $CH_3$ | NMR 268 |
| 103 | " | H | H | |
| 104 | 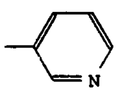 | H | $CH_3$ | |
| 105 | " | H | H | |
| 106 | 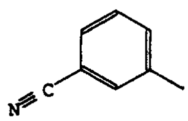 | H | $CH_3$ | |
| 107 | 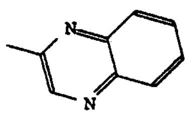 | H | H | |
| 108 | 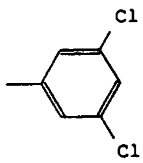 | H | $CH_3$ | |
| 109 | 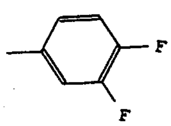 | H | H | |
| 110 | 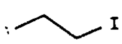 | H | $CH_3$ | |

In der Tabelle I bedeutet das Symbol "״" die Wiederholung eines Substituenten.

Nachfolgend sind die 1H-NMR-Spektren ausgewählter Verbindungen der vorstehenden Tabelle I aufgeführt.

Beispiel 2

$^1$H-NMR (DMSO-d6): δ = 7.81 - 7.09 (7H, m, aryl- und thiophen-H); 5.06, 4.81 (2H, m, N-$CH_2$); 4.34 (1H, qu, J = 7Hz, CH-$CH_3$); 3.34 (2H, m, N-$CH_2CH_2$); 2.59 (3H, s, $CH_3$-triazol); 2.27, 1.60 (2H, m, N-$CH_2CH_2$-); 1.89 (3H, d, J = 7Hz, CH-$CH_3$).

Beispiel 3

$^1$-HNMR (DMSO-d6): δ = 7.83; 7.02; 6.63 (3H, 3 m, Furan-H); 7.59 - 7.30 (4H, m, aryl-H; 5.09; 4.77 (2H, m, N-$CH_2$); 4.26 (1H, qu, J = 7Hz, CH-$CH_3$); 4.02; 3.36 (2H, m, N-$CH_2$-$CH_2$-); 2.59 (3H, s, $CH_3$-triazol); 2.27; 1.60 (2H, m, N-$CH_2CH_2$-); 1.88 (3H, d, J = 7Hz, CH-$CH_3$)-

Beispiel 20

$^1$H-NMR (DMSO-d6): δ = 7.53 - 7.12 (8H, m, aryl-H); 4.97, 4.65 (2H, m, N-$CH_2$); 4.34 (1H, qu, J = 7Hz, CH-$CH_3$) 3.33 (2H, m, N-$CH_2CH_2$-); 2.58 (3H, s, $CH_3$-triazol); 2.33 (3H, s, $CH_3$-aryl); 2.19, 1.49 (2H, m, N-$CH_2CH_2$-); 1.88 (3H, d, J = 7Hz, CH-$CH_3$).

Beispiel 25

$^1$H-NMR (DMSO-d6): δ = 7.55 - 7.19 (8H, m, aryl-H); 4.96, 4.68 (2H, m, N-$CH_2$); 4.34 (1H, qu, J = 7Hz, CH-$CH_3$); 3.33 (2H, m, N-$CH_2CH_2$); 2.58 (3H, s, $CH_3$-triazol); 2.20, 1.51 (2H, m, N-$CH_2CH_2$-); 1.88 (3H, d, J = 7Hz, CH-$CH_3$); 1.29 (9 H, s, C-$(CH_3)_3$).

Beispiel 29

$^1$H-NMR (DMSO-d6): δ = 7.57 - 6.91 (8H, m, aryl-H); 4.86, 4.68 (2H, m, N-$CH_2$); 4.33 (1H, qu, J = 7Hz, CH-$CH_3$); 3.78 (3H, s, $OCH_3$); 3.32 (2H, m, N-$CH_2CH_2$-); 2.58 (3H, s, $CH_3$-triazol); 2.20, 1.51 (2H, m, N-$CH_2CH_2$); 1.88 (3H, d, J. = 7Hz, CH-$CH_3$).

Beispiel 57

$^1$H-NMR (DMSO-d6): δ = 7.58 - 7.16 (8H, m, aryl-H); 4.96, 4.65 (2H, m, N-$CH_2$); 4.34 (1H, qu, J = 7Hz, CH-$CH_3$); 3.32 (2H, m, N-$CH_2CH_2$-); 2.63 (2H, qu, J = 7.5 Hz, -$CH_2$-$CH_3$); 2.58 (3H, s, $CH_3$-triazol); 2.19, 1.51 (2H, m, N-$CH_2CH_2$-); 1.88 (3H, d, J = 7Hz, CH-$CH_3$); 1.18 (3H, t, J = 7.5 Hz, -$CH_2$-$CH_3$).

Beispiel 69

$^1$H-NMR (DMSO-d6): δ = 8.60; 7.24 (4H, m, pyridin-H); 7.50 - 7.28 (4H, m, aryl-H); 4.84; 4.67 (2H, m, N-$CH_2$); 4.86 (1H, qu, J = 7Hz, CH-$CH_3$);
3.61; 3.40, (2H, m, N-$CH_2$-$CH_2$-); 2.58 (2H, s, triazol-H); 2.21; 1.67 (2H, m, N-$CH_2CH_2$-); 1.93 (3H, d,, J = 7Hz, CH-$CH_3$).

Beispiel 73

$^1$H-NMR ($CDCl_3$): δ = 7.53 - 7.19 (4H, m, aryl-H); 4.99; 4.55 (2H, m, N-$CH_2$-); 4.28 (1H, qu, J = 7Hz, CH-$CH_3$); 3.86; 3.42 (2H, m, N-$CH_2CH_2$-); 2.68 (3H, s, $CH_3$-triazol); 2.13 (3H, d, J = 7Hz, CH-$CH_3$); 2.15; 1.75 (2H, m, N-$CH_2CH_2$); 1.25 (9H, s, $C(CH_3)_3$).

Beispiel 82

$^1$H-NMR (DMSO-d6): δ = 7.58 - 7.16 (8H, m, aryl-H); 4.96, 4.65 (2H, m, N-$CH_2$); 4.34 (1H, qu, J = 7Hz, CH-$CH_3$); 3.32 (2H, m, N-$CH_2CH_2$-); 2.63 (2H, qu, J = 7.5 Hz, -$CH_2$-$CH_3$); 2.58 (3H, s, $CH_3$-triazol); 2.19, 1.51 (2H, m, N-$CH_2CH_2$-); 1.88 (3H, d, J = 7Hz, CH-$CH_3$); 1.18 (3H, t, J = 7.5 Hz, -$CH_2$-$CH_3$).

Beispiel 86

$^1$H-NMR ($CDCl_3$): δ = 7.57 - 7.19 (8H, m, aryl-H); 5.06, 4.65 (2H, m, N-$CH_2$); 4.28 (1H, qu, J = 7Hz, CH-$CH_3$); 3.37 (2H, m, N-$CH_2CH_2$-); 2.68 (3H, s, $CH_3$-triazol; 2.18, 1.73 (2H, m, N-$CH_2$-$CH_2$-); 2.12 (3H, d, J = 7Hz, CH-$CH_3$).

Beispiel 89

$^1$H-NMR(DMS0-d6): δ = 8.00 - 7.31 (8H, m, aryl-H); 5.02, 4.70 (2H, m, N-$CH_2$); 4.33 (1H, qu, J = 7Hz, CH-$CH_3$); 3.32 (2H, m, N-$CH_2CH_2$); 2.61 (3H, s, $CH_3$-triazol), 2.20, 1.49 (2H, m, N-$CH_2CH_2$-); 1.88 (3H, d, J = 7Hz, CH-$CH_3$).

Beispiel 92

$^1$H-NMR ($CDCl_3$): δ = 7.53 - 7.10 (8H, m, aryl-H); 5.06, 4.67 (2H, m, N-$CH_2$-); 4.30 (1H, qu, J = 7Hz, CH-$CH_3$); 3.64, 3.39 (2H, m, N-$CH_2CH_2$-); 2.68 (3H, s, $CH_3$-triazol); 2.50 (2H, d, J = 6Hz, $CH_2$-CH); 2.21, 1.87 (2H, m, N-$CH_2CH_2$-); 2.12 (3H, d, J = 7Hz, CH-$CH_3$); 1.89 (1H, m, CH$(CH_3)_2$); 0.90 (6H, d, J = 7Hz, CH-$(CH_3)_2$).

Beispiel 97

$^1$H-NMR (DMSO-d6): δ = 7.89 - 7.28 (8H, m, aryl-H); 5.06, 4.70 (2H, m, N-$CH_2$); 4.34 (1H, qu, J = 7Hz, CH-$CH_3$); 3.33 (2H, m, N-$CH_2CH_2$-); 2.62 (3H, s, $CH_3$-triazol); 2.20, 1.49 (2H, m, N-$CH_2CH_2$-); 1.88 (3H, d, J = 7Hz, CH-$CH_3$).

In Analogie zu dem beschriebenen Synthese-Verfahren B so wie den ausführlich beschriebenen Beispielen 3 und 4 können beispielsweise Verbindungen folgender Struktur hergestellt werden:

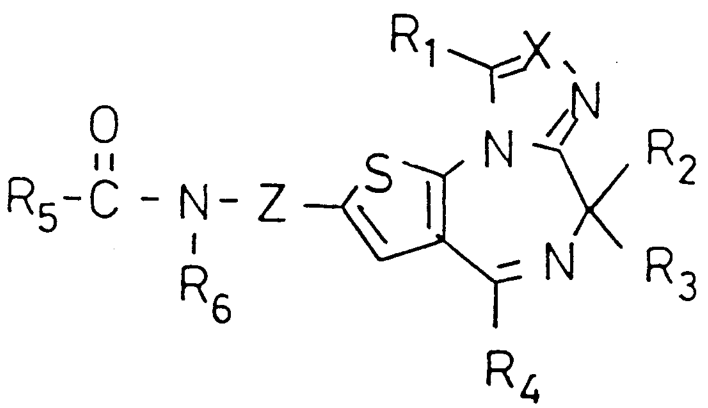

worin
$R_1$ Methyl, $R_3$ Wasserstoff
$R_4$ ortho-Chlorphenyl und $R_5$ 4-Chlorphenyl bedeuten und X, Z, $R_2$ und $R_6$ wie in der Tabelle angegegeben sind.

| Z | $R_2$ | $R_6$ | X |
|---|---|---|---|
| $-CH_2-$ | $-CH_3$ | Et | N |
| $-CH_2-$ | $-CH_3$ | Pr | N |
| $-(CH_2)_3$ | $-CH_3$ | Me | N |
| $-(CH_2)_3-$ | $-CH_3$ | Me | H |
| $-(CH_2)_3-$ | -H | Pr | CH |
| $-(CH_2)_7-$ | -H | Me | N |
| $-(CH_2)_7-$ | $CH_3$ | Me | N |
| $-(CH_2)_7-$ | $-CH_3$ | Me | CH |
| Et = Ethyl | | | |
| Pr = n-Propyl | | | |
| Me = Methyl | | | |

Galenische Zubereitungen

Beispiel a

Tabletten, enthaltend 10 mg Substanz B

| Zusammensetzung: | |
|---|---|
| Substanz B | 10,0 mg |
| Maisstärke | 57,0 mg |
| Milchzucker | 48,0 mg |
| Polyvinylpyrrolidon | 4,0 mg |
| Magnesiumstearat | 1,0 mg |
| | 120,0 mg |

Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mmMaschenweite gedrückt und bei ca. 45°C getrocknet. Das trockne Granulat wird durch ein Sieb mit 1,0 mmMaschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten.
Tablettengewicht: 120 mg
Substanz B = 3-(4-Chlorbenzoyl)-6-(2-chlorphenyl)-8,11-dimethyl-2,3,4,5-tetrahyro-4H-pyrido-[4,3 : 4',5']thieno[3,2-f][1,2,4]-triazolo[4,3-a]-1,4-diazepin

Beispiel b

Dragées, enthaltend 5 mg Substanz B

| Zusammensetzung: | |
|---|---|
| Substanz B | 5,0 mg |
| Maisstärke | 41,5 mg |
| Milchzucker | 30,0 mg |
| Polyvinylpyrrolidon | 3,0 mg |
| Magnesiumstearat | 0,5 mg |
| | 80,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.
Dragéegewicht: 130 mg

Beispiel c

Tabletten, enthaltend 50 mg Substanz B

| Zusammensetzung: | |
|---|---|
| Substanz B | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:

Die Substanz B, Calciumphosphat, Milchzucker und Maisstärke werden mit einer wässerigen Polyvinylpyrrolidonlösung gleichmäßig befeuchtet. Die Masse wird durch ein Sieb mit 2 mm Maschenweite gegeben, im Umlufttrockenschrank bei 50 ° C getrocknet und erneut gesiebt. Nach Zumischen des Schmiermittels wird das Granulat auf einer Tablettiermaschine gepreßt.

Beispiel d

Kapseln, enthaltend 50 mg Substanz B

| Zusammensetzung: | |
|---|---|
| Substanz B | 50,0 mg |
| Maisstärke | 268,5 mg |
| Magnesiumstearat | 1,5 mg |
| | 320,0 mg |

Herstellung:

Substanz B und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel e

Suppositorien, enthaltend 50 mg Substanz B

| Zusammensetzung: | |
|---|---|
| Substanz B | 50 mg |
| Adeps solidus | 1.650 mg |
| | 1.700 mg |

Herstellung:

Das Hartfett wird geschmolzen. Bei 40 ° C wird die gemahlene Wirksubstanz homogen dispergiert. Es wird auf 38 ° C abgekühlt und in schwach vorgekühlte Suppositorienformen ausgegossen.

Beispiel f

Orale Suspension, enthaltend 50 mg Substanz B pro 5 ml

| Zusammensetzung: | |
|---|---|
| Substanz B | 50 mg |
| Hydroxyethylcellulose | 50 mg |
| Sorbinsäure | 5 mg |
| Sorbit 70%ig | 600 mg |
| Glycerin | 200 mg |
| Aroma | 15 mg |
| Wasser ad | 5 ml |

Herstellung:

Destilliertes Wasser wird auf 70 °C erhitzt. Hierin wird unter Rühren Hydroxyethylcellulose gelöst. Nach Zugabe von Sorbitlösung und Glycerin wird auf Raumtemperatur abgekühlt. Bei Raumtemperatur werden Sorbinsäure, Aroma und Substanz B zugegeben. Zur Entlüftung der Suspension wird unter Rühren evakuiert.

Selbstverständlich lassen sich die übrigen erfindundungsgemäßen PAF-Antagonisten in den für sie geeigneten Dosierungen in die üblichen galenischen Zubereitungen einarbeiten.

**Patentansprüche**

**1.** Neue Hetrazepine der allgemeinen Formel I entsprechen der allgemeinen Formel I,

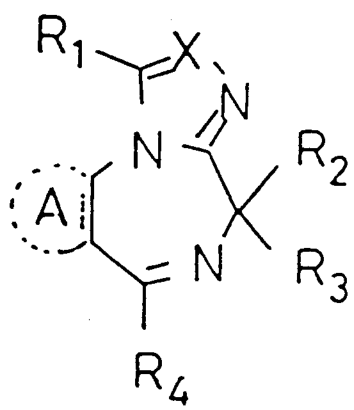

worin

- $R_1$ Wasserstoff, Halogen, eine verzweigte oder unverzweigte Alkylgruppe mit 1 bis 4 Kohlenstoffatom(en), bevorzugt Methyl, die gegebenenfalls durch Hydroxy oder Halogen substituiert sein kann, eine Cyclopropylgruppe, eine Cyclobutylgruppe;
- $R_2$ Wasserstoff, Methyl, Trifluormethyl oder Hydroxymethyl;
- $R_3$ Wasserstoff, Methyl, Trifluormethyl oder Hydroxymethyl;
- $R_4$ Phenyl, wobei der Phenylring ein- oder mehrfach, bevorzugt in 2-Stellung, durch Methyl, bevorzugt Halogen, besonders bevorzugt Chlor oder Brom, Nitro, Alkoxy, bevorzugt Methoxy und/oder Trifluormethyl substituiert sein kann, Pyridyl oder Thienyl, das gegebenenfalls durch $C_1$-$C_4$-Alkyl oder Halogen substituiert sein kann;
- X Stickstoff oder C-H;
- A ein Rest der Formel

a)

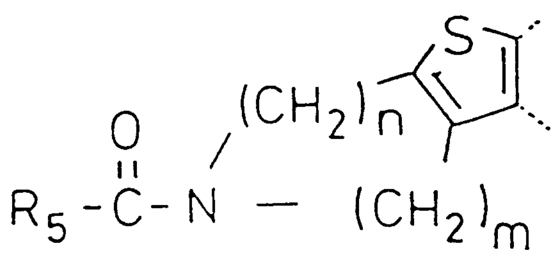

mit
n = 1, 2 oder 3
m = 1, 2 oder 3
und m + n = 2, 3 oder 4;
b)

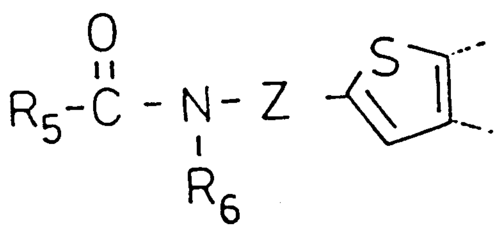

oder
c)

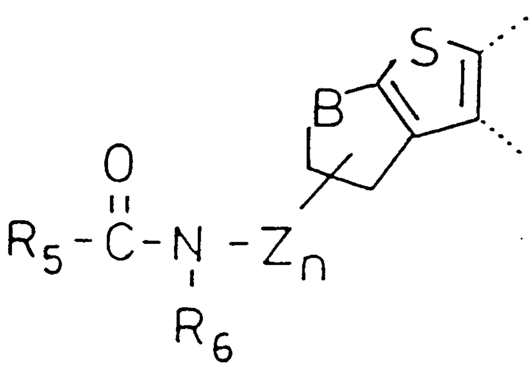

mit B = $CH_2$ oder $-CH_2-CH_2-$

worin

- $R_5$ eine gegebenenfalls substituierte Alkylgruppe mit 1 bis 8, bevorzugt 1 - 4 C-Atomen, eine gegebenenfalls substituierte Arylgruppe, $CH_2$-Aryl, $CH_2$-$CH_2$-Aryl,
- $R_6$ Wasserstoff oder eine gegebenenfalls substituierte Alkylgruppe mit 1 bis 8, bevorzugt 1 - 4 C-Atomen; eine gegebenenfalls substituierte Benzylgruppe und
- Z eine Alkylgruppe mit 1 bis 8 C-Atomen
- $Z_n$ eine Alkylgruppe mit 1 bis 18 C-Atomen oder eine Einfachbindung

bedeuten können,

gegebenenfalls in Form ihrer Racemate, ihrer Enantiomeren, ihrer Diastereomeren und ihrer Gemische.

**2.** Neue Hetrazepine der allgemeinen Formel I

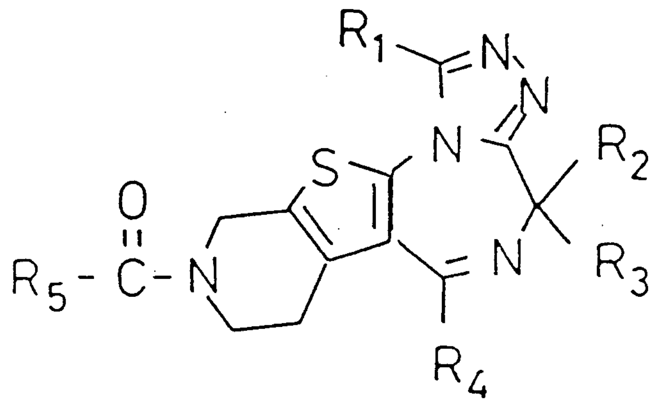

Ia

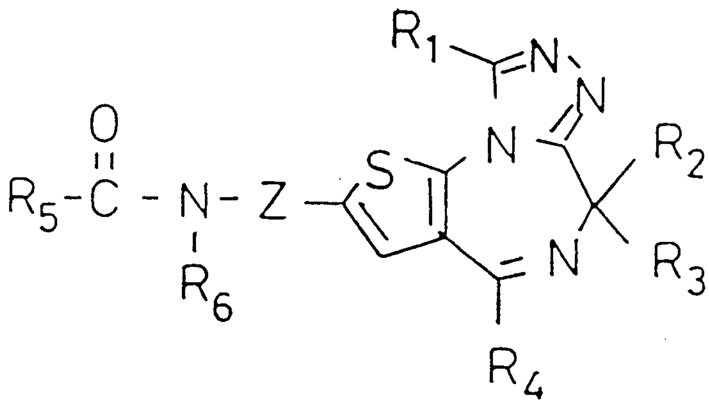

Ib

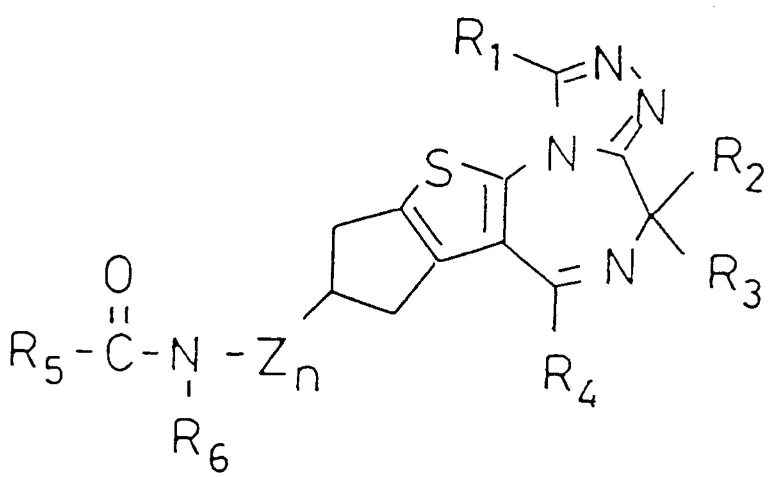

Ic

oder

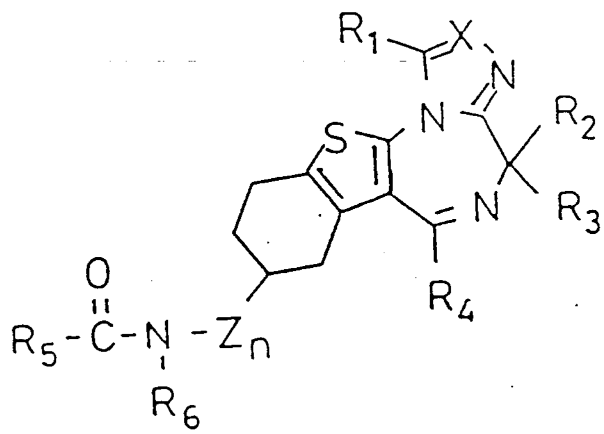

Id

worin

- $R_1$ $CH_3$;
- $R_2$ Wasserstoff oder Methyl;
- $R_3$ Wasserstoff oder Methyl;
- $R_4$ ortho-Chlorphenyl;
- $R_5$ $C_1$-$C_4$-Alkyl, gegebenenfalls substituiertes Phenyl, gegebenenfalls substituiertes -$CH_2$-Phenyl, gegebenenfalls substituiertes Thiophen, gegebenenfalls substituiertes Furan, gegebenenfalls substituiertes Pyridin;
- $R_6$ Wasserstoff oder $C_1$-$C_4$-Alkyl;
- Z eine lineare Alkylgruppe mit 1-4 C-Atomen, bevorzugt $CH_2$;
- $Z_n$ eine lineare Alkylgruppe mit 1-4 C-Atomen oder eine Einfachbindung;

bedeuten können, - gegebenenfalls in Form ihrer Racemate, ihrer Enantiomeren, ihrer Diastereomeren und ihrer Gemische.

**3.** Neue Hetrazepine der allgemeinen Formel Ia

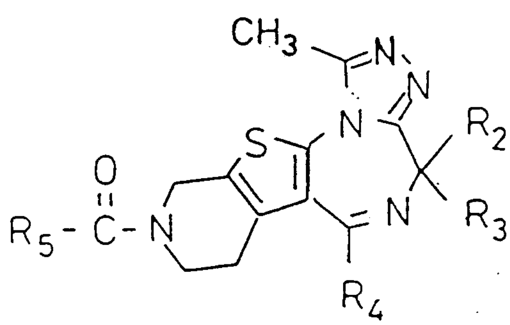

Ia

worin

- $R_2$ Wasserstoff oder Methyl;
- $R_3$ Wasserstoff;
- $R_4$ ortho-Chlorphenyl;
- $R_5$ einen Rest

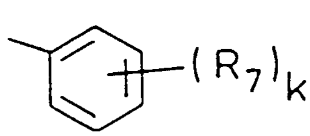 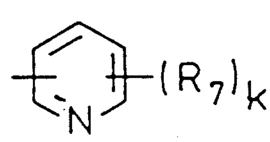

worin

$R_7$ Wasserstoff, Halogen, Hydroxy, Nitro, Cyano, Trifluormethyl, gegebenenfalls durch Halogen oder Hydroxy substituiertes, verzweigtes oder unverzweigtes Alkyl mit 1 bis 8 - bevorzugt 1 bis 4 - Kohlenstoffatom(en), gegebenenfalls durch Halogen oder Hydroxy substituiert, gegebenenfalls substituiertes $C_3$-$C_6$-Cycloalkyl, gegebenenfalls substituiertes, unverzweigtes oder verzweigtes Alkoxy mit 1 bis 8 bevorzugt 1 bis 4 Kohlenstoffatom(en),
$C_1$-$C_4$-Alkyl-S-, HS-, Amino,
$C_1$-$C_6$-Alkylamino,
$C_1$-$C_6$-Dialkylamino,
k = 1, 2 oder 3, wobei bei k > 1 $R_4$ gleich oder verschieden sein kann;

$R_5$ einen Rest

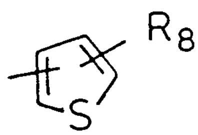

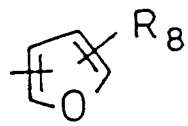

worin

$R_8$ Wasserstoff, Halogen oder $C_1$-$C_4$-Alkyl bedeuten können,

gegebenenfalls in Form ihrer Racemate, ihrer Entantiomeren, ihrer Diastereomeren und ihrer Gemische.

**4.** Verfahren zur Herstellung von Verbindungen der allgemeinen Formel I - wie in einem der Ansprüche 1 bis 3, dadurch gekennzeichnet, daß man ein reaktives Säurederivat der Formel

$R_5CO$ - Y

mit Y = Halogen, OH, oder eine andere reaktive Austrittsgruppe mit einem Amin der allgemeinen Formel

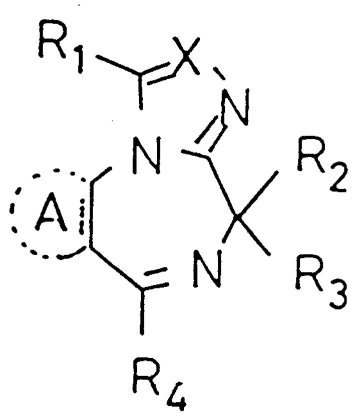

worin A einen Rest

a)

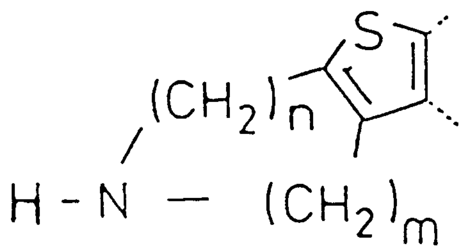

mit
n = 1, 2 oder 3
m = 1, 2 oder 3
und m + n = 2, 3 oder 4;
b)

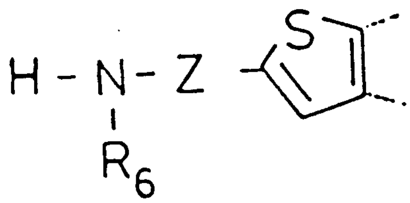

oder
c)

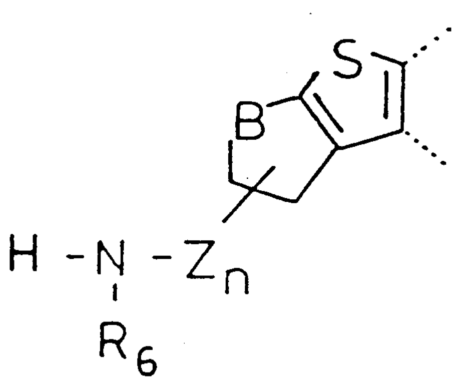

bedeutet, umsetzt.

5. Pharmazeutische Zubereitung, gekennzeichnet durch einen Gehalt an einer Verbindung nach einem der Ansprüche 1 bis 5.

6. Verwendung von Verbindungen nach einem der Ansprüche 1 bis 5 bei der Herstellung von pharmazeutischen Zubereitungen zur Behandlung von Erkrankungen, die durch endogen gebildetes PAF induziert werden.

7. Verwendung von Verbindungen nach einem der Ansprüche 1 bis 5 als Arzneimittel.

Europäisches Patentamt

# EUROPÄISCHER RECHERCHENBERICHT

Nummer der Anmeldung

EP 92 10 3739

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 367 110 (EISAI CO., LTD.)<br>* Ansprüche; Beispiele 6-9, 34, 35, 37, 43-46 * | 1-7 | C07D495/14<br>C07D495/22<br>A61K31/55<br>//(C07D495/14, 333:00,249:00, 243:00)<br>(C07D495/14, 333:00,243:00, 235:00)<br>(C07D495/22, 333:00,249:00, 243:00,221:00) |
| D,X | EP-A-0 328 924 (YOSHITOMI PHARMACEUTICAL INDUSTRIES, LTD.)<br>* Seite 13, Verbindung 10; Seite 16, Verbindungen 41-44; Seite 17, Verbindungen 47, 48; Zusammenfassung * | 1,2,5-7 | |
| D,X | EP-A-0 230 942 (BOEHRINGER INGELHEIM KG)<br>* Seite 42, Verbindungen 35-38, 41, 42; Ansprüche 1, 8-10 * | 1,2,5-7 | |
| A,D | EP-A-0 407 955 (BOEHRINGER INGELHEIM KG ET AL.)<br>* Ansprüche 1,4-6; Beispiele * | 1,2,5-7 | |
| A,D | EP-A-0 254 245 (BOEHRINGER INGELHEIM KG)<br>* Ansprüche 1,8-10; Beispiele * | 1,2,5-7 | |
| A | EP-A-0 338 993 (SANDOZ AG ET AL.)<br>* Ansprüche 1,6-8 * | 1,5-7 | |

RECHERCHIERTE SACHGEBIETE (Int. Cl.5)

C07D

Der vorliegende Recherchenbericht wurde für alle Patentansprüche erstellt

| Recherchenort | Abschlußdatum der Recherche | Prüfer |
|---|---|---|
| BERLIN | 18 MAI 1992 | HASS C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)